(19) Europäisches Patentamt
European Patent Office
Office européen des brevets

(11) Numéro de publication : **0 356 413 B1**

# (12) FASCICULE DE BREVET EUROPEEN

(45) Date de publication du fascicule du brevet : **29.01.92 Bulletin 92/05**

(51) Int. Cl.5 : **C07D 251/18,** A61K 31/535

(21) Numéro de dépôt : **89870120.6**

(22) Date de dépôt : **28.07.89**

(54) **2-Amino-4-morpholino-6-propyl-1,3,5-triazines.**

Le dossier contient des informations techniques présentées postérieurement au dépôt de la demande et ne figurant pas dans le présent fascicule.

(30) Priorité : **16.08.88 GB 8819493**

(43) Date de publication de la demande : **28.02.90 Bulletin 90/09**

(45) Mention de la délivrance du brevet : **29.01.92 Bulletin 92/05**

(84) Etats contractants désignés : **AT BE CH DE ES FR GB GR IT LI LU NL SE**

(56) Documents cités :
**DE-A- 2 226 474**
**FR-A- 2 262 512**
**US-A- 3 549 755**

(73) Titulaire : **UCB S.A.**
**Avenue Louise 326, Bte 7**
**B-1050 Bruxelles (BE)**

(72) Inventeur : **Cossement, Eric**
**105, rue des Echevins**
**B-1050 Bruxelles (BE)**
Inventeur : **Gobert, Jean**
**120, rue du Cornet**
**B-1040 Bruxelles (BE)**
Inventeur : **Boydens, Roland**
**235, rue Roosendael**
**B-1190 Bruxelles (BE)**
Inventeur : **Mathieu, Jacques**
**44, avenue Heydenberg**
**B-1200 Bruxelles (BE)**

(74) Mandataire : **Dusseldorp, Raymond et al**
**U.C.B. S.A. Département D.T.B. 326, avenue Louise, Bte 7**
**B-1050 Bruxelles (BE)**

EP 0 356 413 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à de nouvelles 2-amino-4-morpholino-6-propyl-1,3,5-triazines, à leurs sels d'addition d'acides non toxiques pharmaceutiquement acceptables ainsi qu'à des procédés pour les préparer. Elle concerne également les compositions thérapeutiques renfermant lesdits composés.

Selon la demande de brevet japonaise 69688/74, on connaît déjà des 2-amino-1,3,5-triazines substituées en position 4 par un groupe amino, amino mono- ou disubstitué par un radical alkyle, alkényle, cycloalkyle, aryle ou aralkyle, ou par un radical hétérocyclique azoté, et en position 6 par un radical alkyle, aryle ou aralkyle. En particulier, on y décrit la préparation de la 2-amino-4-morpholino-6-propyl-1,3,5-triazine. Selon cette demande de brevet, ces composés possèdent la propriété d'augmenter la sécrétion des corticoïdes et en particulier des glucocorticoïdes.

Par ailleurs, K. WAKABAYASHI KO et al., (Yuki Gosei Kagaku Kyokai Shi, 28, (1970), 252-260 ; Chem.Abstr., 72, (1970), 100653v) ont synthétisé des 2-alkyl-4,6-bis(alkylamino)-1,3,5-triazines dans lesquelles le radical en position 2 contient de 1 à 17 atomes de carbone et peut aussi représenter le groupe trichlorométhyle, tribromométhyle ou 2-chloréthyle, tandis que les radicaux en positions 4 et 6 sont identiques et peuvent être substitués par un groupe hydroxyle, ou bien, représentent un hétérocycle azoté tel que le radical pipéridino ou morpholino. Un composé représentatif de cette famille est la 2-méthyl-4,6-dimorpholino-1,3,5-triazine. Ces composés sont étudiés pour leur activité herbicide ainsi que pour leur propriété d'inhiber la transformation de l'ammoniac en nitrate. Toutefois, il n'y est pas fait mention de propriétés pharmacologiques.

On a maintenant trouvé de nouvelles 2-amino-4-morpholino-6-propyl-1,3,5-triazines qui possèdent des propriétés pharmaceutiques de valeur et en particulier la propriété de potentialiser les effets cholinergiques centraux et périphériques provoqués par un agent cholinomimétique tel que, par exemple, l'oxotrémorine, alors que ces composés ne présentent pas d'effet cholinergique propre. De plus, on a trouvé également que ces composés ont la propriété avantageuse d'atténuer les effets résultant d'un hypofonctionnement cholinergique induit par un antagoniste cholinergique tel que, par exemple, la scopolamine. Le système cholinergique est largement impliqué dans les phénomènes de mémorisation et d'apprentissage. Ainsi, par exemple, l'administration à des sujets jeunes d'un agent anticholinergique tel que la scopolamine fait apparaître des déficiences de la mémoire semblables à celles observées chez des sujets âgés. Inversement, des agents cholinergiques, comme la physostigmine, sont capables de combattre l'amnésie résultant de l'administration d'agents anticholinergiques (S.D. GLICK et al., Behavioral Biology, 7, (1972), 245-254 ; U. SCHINDLER et al., Drug Develop.Res. 4, (1984), 567-576). Par ailleurs, il est actuellement bien établi qu'une des caractéristiques le plus fréquemment associées aux démences est précisément une dégradation du système cholinergique (Cellular and molecular basis of cholinergic function. Ed. M.J. DOWDALL and J.N. HAWTHORNE, 1987, Chapitre 99 : art. de A. NORDBERG et al.). C'est pourquoi, ces composés peuvent être utilisés pour le traitement des troubles cognitifs et comportementaux associés à l'âge et aux syndromes démentiels. En particulier, ils trouvent une application dans le traitement des troubles associés à la maladie d'Alzheimer, aux démences séniles de type Alzheimer et à toute pathologie cognitive évolutive (C.G. GOTTFRIES, Psychopharmacology, 86, (1985), 245-252 ; C.G. GOTTFRIES, Neurobiology of Ageing, 4, (1983), 261-271).

Plus particulièrement, la présente invention se rapporte à de nouvelles 2-amino-4-morpholino-6-propyl-1,3,5-triazines répondant à la formule générale

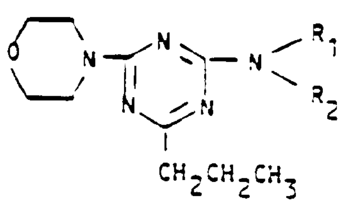

(I)

dans laquelle

$R_1$ représente un atome d'hydrogène, un radical alkyle, phénylalkyle ou acétyle,

$R_2$ représente un groupe hydroxyle, un radical hydroxyalkyle, alkoxyalkyle, dialkylamino, phénylhydroxyalkyle, (hydroxycycloalkyl)alkyle, alkanoyloxyalkyle, benzoyloxyalkyle, phénylacétyloxyalkyle ou aminocarbonyloxyalkyle, un groupe $COR_3$ dans lequel $R_3$ représente un radical alkyle, phényle, halogénophényle, alkylphényle, alkoxyphényle, phénylalkyle ou phénoxy, ou un groupe $CONR_4R_5$ dans lequel $R_4$ et $R_5$ représentent de l'hydrogène ou un radical alkyle, ou

$R_1$ et $R_2$ forment conjointement avec l'atome d'azote auquel ils sont attachés un radical pyrrolidino ou

pipéridino substitué par un radical hydroxyalkyle, les radicaux alkyle, alkoxy et alkanoyloxy ayant de 1 à 4 atomes de carbone et les radicaux cycloalkyle ayant de 4 à 6 atomes de carbone,

avec la restriction que lorsque $R_1$ représente le radical acétyle, $R_2$ représente un radical acétoxyalkyle, ainsi qu'à leurs sels d'addition d'acides non toxiques pharmaceutiquement acceptables.

Comme exemples de radical hydroxyalkyle, on citera les radicaux 2-hydroxyéthyle, 3-hydroxypropyle, 2-hydroxypropyle, 2-hydroxybutyle, 1-(hydroxyméthyl)propyle, 2,3-dihydroxypropyle, 2-hydroxy-1,1-bis(hydroxyméthyl)éthyle, etc.

Comme exemple de radical phényl-hydroxyalkyle, on citera le radical 2-hydroxy-1-(hydroxyméthyl)-2-phényléthyle, etc.

Comme exemple de radical (hydroxy-cycloalkyl)alkyle, on citera le radical (1-hydroxycyclohexyl)méthyle, etc.

Comme exemples de radical alkanoyloxyalkyle, on citera les radicaux 2-(acétoxy)éthyle, 2-(isobutyryloxy)éthyle, etc.

Comme exemple de radical pyrrolidino ou pipéridino substitué par un radical hydroxyalkyle, on citera les radicaux 2-hydroxyméthylpyrrolidino, 2-hydroxyméthyl-pipéridino, etc.

Les composés préférés de la présente invention sont les 2-amino-4-morpholino-6-propyl-1,3,5-triazines répondant à la formule générale I, dans laquelle

$R_1$ représente un atome d'hydrogène ou un radical alkyle,

$R_2$ représente un groupe hydroxyle, un radical hydroxyalkyle, alkoxyalkyle, dialkylamino, alkanoyloxyalkyle ou un groupe $COR_3$ dans lequel $R_3$ représente un radical phényle substitué ou non par un atome d'halogène ou un radical alkyle ou alkoxy, les radicaux alkyle, alkoxy et alkanoyloxy ayant de 1 à 4 atomes de carbone,

ainsi que leurs sels d'addition d'acides non toxiques pharmaceutiquement acceptables.

Les composés particulièrement préférés conformes à l'invention sont :

- le 2-[(4-morpholino-6-propyl-1,3,5-triazin-2-yl)amino]-éthanol,
- la 2-(hydroxyamino)-4-morpholino-6-propyl-1,3,5-triazine,
- la 2-[(2-méthoxyéthyl)amino]-4-morpholino-6-propyl-1,3,5-triazine,
- le (S)-3-[(4-morpholino-6-propyl-1,3,5-triazin-2-yl)amino]-2-propanol,
- le (R)-3-[(4-morpholino-6-propyl-1,3,5-triazin-2-yl)amino]-2-propanol,
- la 2-(2,2-diméthylhydrazino)-4-morpholino-6-propyl-1,3,5-triazine,
- le chlorhydrate de N-(4-morpholino-6-propyl-1,3,5-triazin-2-yl)-benzamide,
- le chlorhydrate de N-méthyl-N-(4-morpholino-6-propyl-1,3,5-triazin-2-yl)-benzamide,
- la 2-[[2-(acétoxy)éthyl]amino]-4-morpholino-6-propyl-1,3,5-triazine.

La présente invention concerne également les sels d'addition d'acides non toxiques pharmaceutiquement acceptables des 2-amino-4-morpholino-6-propyl-1,3,5-triazines de formule I. Comme exemples d'acides pharmaceutiquement acceptables, on peut citer les acides minéraux comme les acides chlorhydrique, bromhydrique, sulfurique, nitrique, phosphorique, etc., et les acides organiques comme les acides acétique, citrique, tartrique, benzoïque, salicylique, maléique, etc.

Lorsque la molécule comporte un atome de carbone asymétrique, les composés de formule I peuvent se présenter soit sous la forme d'un mélange racémique, soit sous la forme de l'un ou l'autre énantiomère. Ces diverses formes entrent également dans le cadre de la présente invention.

Les 2-amino-4-morpholino-6-propyl-1,3,5-triazines conformes à la présente invention peuvent être préparées par l'un ou l'autre des procédés suivants :

(a) Lorsque dans la formule I, $R_1$ représente un atome d'hydrogène, un radical alkyle ou phénylalkyle et $R_2$ représente un groupe hydroxyle, un radical hydroxyalkyle, alkoxyalkyle, dialkylamino, phényl-hydroxyalkyle ou (hydroxy-cycloalkyl)alkyle, ou $R_1$ et $R_2$ forment conjointement avec l'atome d'azote auquel ils sont attachés un radical pyrrolidino ou pipéridino substitué par un radical hydroxyalkyle, on fait réagir la 2-chloro-4-morpholino-6-propyl-1,3,5-triazine de formule II avec une amine de formule $HNR_1R_2$ (III) selon l'équation :

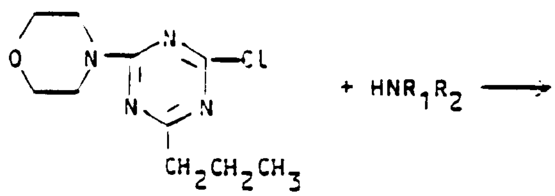


(II) (III) (I)

dans ces formules $R_1$ et $R_2$ ont la signification donnée ci-dessus.

(b) Lorsque dans la formule I, $R_1$ représente un atome d'hydrogène, un radical alkyle ou phénylalkyle et $R_2$ représente un groupe hydroxyle, un radical hydroxyalkyle, alkoxyalkyle, dialkylamino, phényl-hydroxyalkyle ou (hydroxy-cycloalkyl)alkyle, ou $R_1$ et $R_2$ forment conjointement avec l'atome d'azote auquel ils sont attachés un radical pyrrolidino ou pipéridino substitué par un radical hydroxyalkyle, on fait réagir une 2-amino-4-chloro-6-propyl-1,3,5-triazine de formule IV avec une morpholine de formule V, selon l'équation

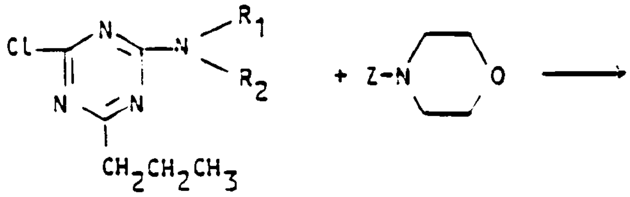

(IV) (V) (I)

dans ces formules $R_1$ et $R_2$ ont la signification donnée ci-dessus et Z représente un atome d'hydrogène ou un radical méthyle.

Les procédés (a) et (b) ci-dessus sont réalisés à température élevée, généralement à la température d'ébullition du solvant employé, et en présence d'une base. Le solvant dans lequel on effectue ces réactions est soit l'amine elle-même, utilisée en excès, soit un solvant organique inerte, de préférence le dioxanne, et dans ce dernier cas, la base utilisée est une base minérale ou organique autre que l'amine mise en oeuvre dans la réaction, par exemple la triéthylamide.

La 2-chloro-4-morpholino-6-propyl-1,3,5-triazine (II) utilisée comme produit de départ est déjà connue (T.TSUJIKAWA et al., Yakugaku Zasshi, 95, (1975), 512-520).

Les composés de départ de formule IV sont préparés, selon les méthodes conventionnelles, en faisant réagir la 2,4-dichloro-6-propyl-1,3,5-triazine avec une amine de formule $HNR_1R_2$ (III) dans laquelle $R_1$ et $R_2$ ont la signification donnée ci-dessus.

(c) Lorsque dans la formule I, $R_1$ représente un atome d'hydrogène, un radical alkyle ou phénylalkyle et $R_2$ représente le groupe $COR_3$ dans lequel $R_3$ est un radical alkyle, phényle, halogénophényle, alkylphényle, alkoxyphényle, phénylalkyle ou phénoxy, les radicaux alkyle et alkoxy ayant de 1 à 4 atomes de carbone, on fait réagir dans des proportions équimolaires une 2-amino-4-morpholino-6-propyl-1,3,5-triazine de formule VI avec un halogénure de $R_3$-carbonyle de formule VII, selon l'équation

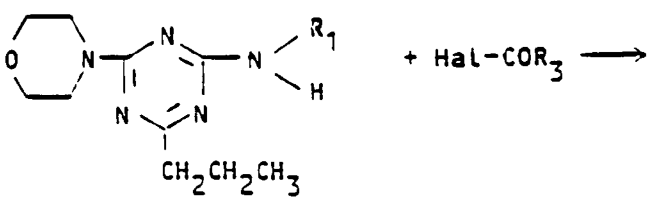


(VI) (VII) (I)

dans ces formules $R_1$ et $R_3$ ont la signification donnée ci-dessus et Hal représente un atome d'halogène, de préférence un atome de chlore.

Cette réaction, connue en soi, est généralement effectuée dans un solvant organique comme, par exemple,

le dichlorométhane, le dichloroéthane ou la pyridine, à une température comprise entre la température ambiante et la température d'ébullition du solvant et en présence d'un accepteur d'acide tel qu'une base organique tertiaire (p.ex. la triéthylamine ou la pyridine) ou une base minérale.

Les composés de départ de formule VI peuvent être préparés selon l'un ou l'autre des deux procédés (a) ou (b) décrits précédemment.

(d) Lorsque dans la formule I, $R_1$ représente un atome d'hydrogène, un radical alkyle ou phénylalkyle et $R_2$ représente un radical alkanoyloxyalkyle, benzoyloxyalkyle ou phénylacétyloxyalkyle, les radicaux alkyle et alkanoyle ayant de 1 à 4 atomes de carbone, on fait réagir dans des proportions équimolaires un (4-morpholino-6-propyl-1,3,5-triazin-2-yl)-aminoalcanol de formule VIII avec un halogénure de $R_6$-carbonyle de formule IX, selon l'équation

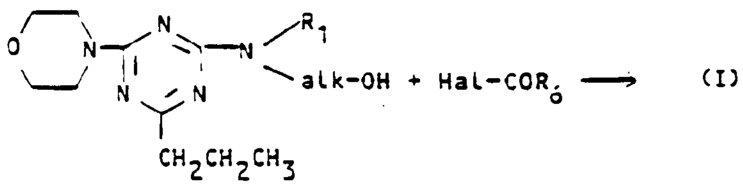

(VIII) (IX)

dans ces formules $R_1$ a la signification donnée ci-dessus, alk représente un radical alkylène en $C_1$-$C_4$, $R_6$ représente un radical alkyle en $C_1$-$C_4$, phényle ou benzyle et Hal représente un atome d'halogène, de préférence un atome de chlore.

Cette réaction, connue en soi, est généralement effectuée dans un solvant organique comme, par exemple, le dichlorométhane, le dichloroéthane ou la pyridine, à une température comprise entre 0°C et 25°C et en présence d'un accepteur d'acide tel qu'une base organique tertiaire (p.ex. la triéthylamine ou la pyridine) ou une base minérale.

Les composés de départ de formule VIII peuvent être préparés selon l'un ou l'autre des deux procédés (a) ou (b) décrits précédemment.

(e) Lorsque dans la formule I, $R_1$ représente un atome d'hydrogène, un radical alkyle ou phénylalkyle et $R_2$ un groupe $CONR_4R_5$ dans lequel $R_4$ et $R_6$ représentent de l'hydrogène ou un radical alkyle, les radicaux alkyles ayant de 1 à 4 atomes de carbone, on fait réagir un (4-morpholino-6-propyl-1,3,5-triazin-2-yl)carbamate de phényle de formule X avec un composé azoté de formule $HNR_4R_5$ (XI), selon l'équation

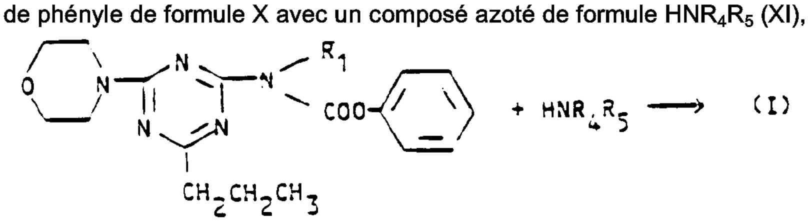

(X) (XI)

dans ces formules $R_1$, $R_4$ et $R_6$ ont la signification donnée ci-dessus. Cette réaction est généralement effectuée dans un solvant inerte tel que le dichlorométhane, et à une température comprise entre – 45°C et + 25°C.

Les composés de départ de formule X peuvent être préparés selon le procédé (c) ci-dessus, en faisant réagir une 2-amino-4-morpholino-6-propyl-1,3,5-triazine de formule VI, dans laquelle $R_1$ a la signification donnée ci-dessus, avec un halogénure de $R_3$-carbonyle de formule VII dans laquelle $R_3$ représente le radical phénoxy et Hal représente un atome d'halogène, de préférence un atome de chlore.

(f) Lorsque dans la formule I, $R_1$ représente un atome d'hydrogène, un radical alkyle ou phénylalkyle, et $R_2$ un radical aminocarbonyloxyalkyle, les radicaux alkyles ayant de 1 à 4 atomes de carbone, on fait réagir de l'ammoniac avec une 2-morpholino-4-(phénoxycarbonyloxyalkylamino)-6-propyl-1,3,5-triazine de formule XII, selon l'équation

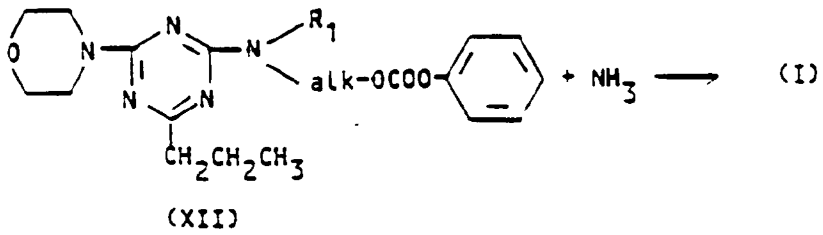

dans ces formules $R_1$ a la signification donnée ci-dessus et alk représente un radical alkylène en $C_1$-$C_4$.

Cette réaction est généralement effectuée dans un solvant inerte tel que le dichlorométhane, et à une température comprise entre – 35°C et – 45°C.

Les composés de départ de formule XII peuvent être préparés selon le procédé (d) ci-dessus, en faisant réagir un (4-morpholino-6-propyl-1,3,5-triazin-2-yl) aminoalcanol de formule VIII arec un halogénoformiate de phényle de formule XIII, selon l'équation

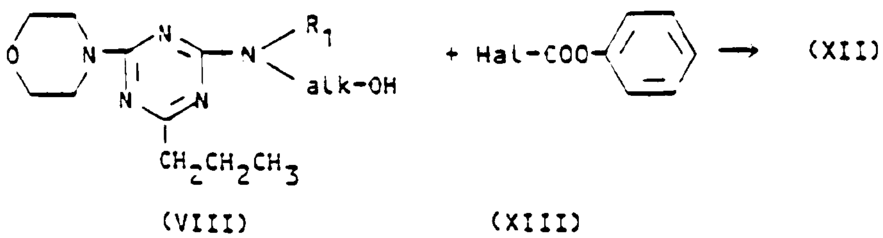

dans ces formules $R_1$ a la signification donnée ci-dessus, alk représente un radical alkylène en $C_1$-$C_4$ et Hal un atome d'halogène, de préférence un atome de chlore.

(g) Lorsque dans la formule I, $R_1$ représente le radical acétyle et $R_2$ un radical acétoxyalkyle dont le radical alkyle possède 1 à 4 atomes de carbone, on fait réagir au moins deux moles d'un halogénure d'acétyle, de préférence le chlorure d'acétyle, avec une mole d'un (4-morpholino-6-propyl-1,3,5-triazin-2-yl)aminoalcanol de formule XIV, selon l'équation

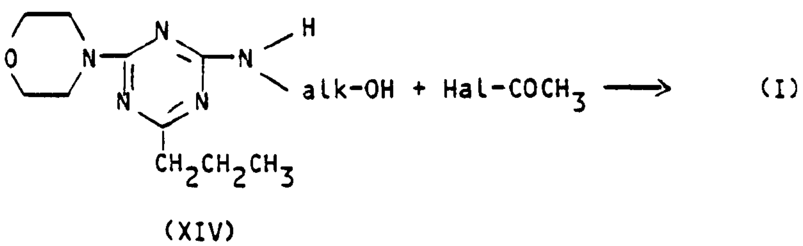

dans ces formules alk représente un radical alkylène en $C_1$-$C_4$ et Hal un atome d'halogène, de préférence un atome de chlore. Cette réaction est effectuée dans un solvant inerte tel que le dichlorométhane, à une température comprise entre la température ambiante et la température de reflux du solvant, et en présence d'une base telle que la triéthylamine.

Les composés de départ de formule XIV peuvent être préparés selon l'un ou l'autre des deux procédés (a) ou (b) décrits précédemment.

Les sels d'addition d'acides non toxiques pharmaceutiquement acceptables peuvent être préparés à partir des 2-amino-4-morpholino-6-propyl-1,3,5-triazines de formule I par des méthodes connues en soi.

Les exemples, qui suivent illustrent la présente invention sans la limiter.

Exemple 1. Préparation selon le procédé (a).

2-[(4-morpholino-6-propyl-1,3,5-triazin-2-yl)amino]-éthanol (composé 1).

On dissout 145,5 g (0,6 mole) de 2-chloro-4-morpholino-6-propyl-1,3,5-triazine dans 600 ml de dioxanne. Dans cette solution on introduit, goutte à goutte et sous une bonne agitation, en 80 minutes environ, une solution de 444 g (7,28 moles) de 2-aminoéthanol dans 600 ml de dioxanne. Ensuite, le mélange réactionnel est chauffé à reflux pendant 7 heures. On refroidit, on décante le chlorhydrate de 2-aminoéthanol qui s'est formé et on élimine le dioxanne sous pression réduite. On dissout le résidu obtenu dans 5 litres d'éther diéthylique. Cette solution est lavée à l'eau et l'eau de lavage est réextraite par du dichlorométhane. Les phases organiques sont réunies et séchées sur sulfate de magnésium. On élimine le solvant sous pression réduite. Le résidu solide obtenu est recristallisé dans un mélange 50 : 50 (v/v) acétate d'éthyle-éther de pétrole. On obtient 139,8 g de 2-[(4-morpholino-6-propyl-1,3,5-triazin-2-yl)amino]-éthanol.
Rendement : 88%. P.F. : 95-99°C.
Analyse pour $C_{12}H_{21}N_5O_2$ en %

| | | | |
|---|---|---|---|
| calculé : | C 53,91 | H 7,92 | N 26,20 |
| trouvé : | 54,30 | 8,10 | 26,13 |

Le 2-[(4-morpholino-6-propyl-1,3,5-triazin-2-yl)amino]-éthanol peut se présenter sous deux formes cristallines différentes selon que le produit a été recristallisé dans le méthanol (P.F. : 97-99°C) ou dans le toluène (P.F. : 89-90°C).

On prépare de la même manière les composés de formule I réunis dans le Tableau I :

**TABLEAU I**

**2-R-4-morpholino-6-propyl-1,3,5-triazines**

| Composé | Substituant R | Mol (1) | Rdt (%) | P.F.(°C) | Analyses | calculé % | trouvé % |
|---|---|---|---|---|---|---|---|
| 2 | NHOH | 1,1 (2) | 51,6 | 93-94 | C | 55,68 | 55,70 |
| | | | | | H | 8,20 | 8,19 |
| | | | | | N | 32,24 | 32,19 |
| 3 | $NHCH_2CH_2CH_2OH$ | 2 | 86,6 | 95-96 | C | 55,51 | 55,02 |
| | | | | | H | 8,18 | 8,26 |
| | | | | | N | 24,91 | 24,42 |
| 4 | $NH-(CH_2)_3-CH_2OH$ | 2 | 74,6 | 79-80 | C | 56,95 | 56,95 |
| | | | | | H | 8,47 | 8,61 |
| | | | | | N | 23,73 | 23,42 |
| 5 | $NHCH_2-CH(OH)-CH_3$ | 3 | 90,0 | 104-105 | C | 55,52 | 55,50 |
| | | | | | H | 8,19 | 8,00 |
| | | | | | N | 24,91 | 23,48 |

**TABLEAU I (suite)**

| Composé | Substituant R | Mol (1) | Rdt (%) | P.F.(°C) | Analyses | calculé % | trouvé % |
|---|---|---|---|---|---|---|---|
| 6 | $NHCH_2-CH(OH)-C_2H_5$ | 1 (3) | 22,0 | 56-57 | C | 56,95 | 56,76 |
| | | | | | H | 8,47 | 8,37 |
| | | | | | N | 23,73 | 23,50 |
| 7 | $NH-CH(C_2H_5)-CH_2OH$ | 2 | 75,4 | 90-91 | C | 56,95 | 56,92 |
| | | | | | H | 8,47 | 8,40 |
| | | | | | N | 23,73 | 23,60 |
| 8 | $NH-C(CH_3)_2-CH_2OH$ | 2 | 32,2 | 85-86 | C | 56,95 | 57,87 |
| | | | | | H | 8,47 | 8,12 |
| | | | | | N | 23,73 | 24,24 |
| 9 | $N(CH_3)-CH_2CH_2OH$ | 2 | 62,6 | 32-33 | C | 55,51 | 55,48 |
| | | | | | H | 8,19 | 8,30 |
| | | | | | N | 24,91 | 24,86 |
| 10 | $NHCH_2CH_2OCH_3$ | 2 | 42,7 | 44-45 | C | 55,52 | 55,60 |
| | | | | | H | 8,19 | 8,20 |
| | | | | | N | 24,91 | 24,99 |
| 11 | $NHCH_2CHOHCH_2OH$ | 2 | 68,7 | 119-120 | C | 52,52 | 52,69 |
| | | | | | H | 7,74 | 7,76 |
| | | | | | N | 23,57 | 23,60 |
| 12 | $NH-C(CH_2OH)_3$ | 2 | 61,0 | 110-111 | C | 51,37 | 50,94 |
| | | | | | H | 7,64 | 7,53 |
| | | | | | N | 21,41 | 21,16 |
| 13 | $NH-C(CH_2OH)_2-CH_3$ | 2 | 48,0 | 121-122 | C | 54,02 | 54,13 |
| | | | | | H | 8,04 | 8,00 |
| | | | | | N | 22,5 | 22,2 |
| 14 | $N(CH_2C_6H_5)C_2H_4OH$ | 2 | 50 | 57-58 | C | 63,86 | 64,60 |
| | | | | | H | 7,56 | 7,76 |
| | | | | | N | 19,6 | 19,8 |

TABLEAU I (suite)

| Composé | Substituant R | Mol (1) | Rdt (%) | P.F. (°C) | Analyses | calculé % | trouvé % |
|---|---|---|---|---|---|---|---|
| 15 | $NHCH(CH_2OH)-CHOHC_6H_5$ | 2 | 63 | 107-108 | C | 61,13 | 62,00 |
| | | | | | H | 7,24 | 7,45 |
| | | | | | N | 18,77 | 18,92 |
| 16(4) | $NHCH_2CHOHCH_3$ | 2 | 38 | 97-98 | C | 55,52 | 56,30 |
| | | | | | H | 8,19 | 8,42 |
| | | | | | N | 24,91 | 24,70 |
| 17(5) | $NHCH_2CHOHCH_3$ | 2 | 49 | 94-95 | C | 55,52 | 55,64 |
| | | | | | H | 8,19 | 8,13 |
| | | | | | N | 24,91 | 24,73 |
| 18 | 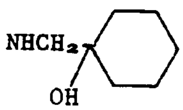 | 2 | 63 | 75-76 | C | 60,90 | 59,83 |
| | | | | | H | 8,66 | 8,73 |
| | | | | | N | 20,99 | 20,48 |
| 19(6) | 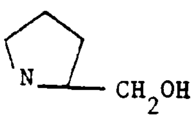 | 2 | 47 | 134-136 | C | 52,40 | 52,48 |
| | | | | | H | 7,57 | 7,40 |
| | | | | | N | 20,38 | 19,83 |
| 20 | $NHN(CH_3)_2$ | 2 | 63,6 | 105-106 | C | 54,14 | 54,39 |
| | | | | | H | 8,97 | 8,81 |
| | | | | | N | 31,58 | 31,65 |

(1) nombre de moles de l'amine par mole de produit de départ;
(2) en présence de 2 moles de triéthylamine;
(3) en présence de 1 mole de triéthylamine;
(4) énantiomère de configuration S; $[\alpha]_D^{25}$ = + 15,3 (C = 1, méthanol);
(5) énantiomère de configuration R; $[\alpha]_D^{25}$ = - 16,1 (C = 1, méthanol);
(6) chlorhydrate.

La 2-chloro-4-morpholino-6-propyl-1,3,5-triazine utilisée comme produit de départ pour la synthèse des composés cités dans cet exemple, a été préparée selon la méthode décrite par T.TSUJIKAWA et al., (Yakugaku Zasshi, 95, (1975), 512-520) à partir de la 2,4-dichloro-6-propyl-1,3,5-triazine. Ce dernier composé a été préparé selon le procédé de R. HIRT et al., (Helv. Chim. Acta, 33, (1950), 1365-69).

Exemple 2. Préparation selon le procédé (b).

a) Obtention du 2-[(4-chloro-6-propyl-1,3,5-triazin-2-yl)amino]-éthanol de départ.

A la température ambiante, on ajoute goutte à goutte 24,4 g (0,4 mole) de 2-aminoéthanol dissous dans 100 ml de dioxanne, à une solution de 38,4 g (0,2 mole) de 2,4-dichloro-6-propyl-1,3,5-triazine dans 100 ml de dioxanne. Après l'addition, on maintient encore l'agitation pendant une nuit. On filtre le chlorhydrate de 2-aminoéthanol et le filtrat est évaporé sous pression réduite. Le résidu obtenu est recristallisé dans un mélange acétate d'éthyle-hexane 50 : 50 (v/v). On obtient 41,5 g de 2-[(4-chloro-6-propyl-1,3,5-triazin-2-yl)amino]-éthanol.
Rendement : 96%. P.F. : 87-88°C.
Analyse pour $C_8H_{13}ClN_4O$ en %

| | | | | |
|---|---|---|---|---|
| calculé : | C 44,34 | H 6,00 | N 25,88 | Cl 16,40 |
| trouvé : | 44,42 | 6,17 | 25,69 | 16,52 |

b) Préparation du 2-[(4-morpholino-6-propyl-1,3,5-triazin-2-yl)amino]-éthanol.

— Variante 1.

On mélange 10,83 g (0,05 mole) de 2-[(4-chloro-6-propyl-1,3,5-triazin-2-yl)amino]-éthanol et 8,7 g (0,10 mole) de morpholine dans 150 ml de dioxanne. La température du mélange s'élève spontanément jusqu'à 41°C. On chauffe ensuite à reflux pendant 30 minutes puis on refroidit et on filtre le chlorhydrate de morpholine qui s'est formé. On évapore le filtrat et on recristallise le résidu dans un mélange 50 : 50 (v/v) acétate d'éthyl-hexane. On obtient 11,8 g de 2-[(4-morpholino-6-propyl-1,3,5-triazin-2-yl)amino]-éthanol.
Rendement : 88,4%. P.F. : 93-94°C.

Le produit, recristallisé dans le méthanol, fond à 97-98°C et est identique au composé 1 préparé à l'exemple 1.

— Variante 2.

On mélange 10,83 g (0,05 mole) de 2-[(4-chloro-6-propyl-1,3,5-triazin-2-yl)amino]-éthanol et 15,15 g (0,15 mole) de N-méthylmorpholine, dans 100 ml de dioxanne. On chauffe le mélange à reflux pendant 20 heures ; ensuite, on refroidit et on élimine le dioxanne sous pression réduite. Le résidu est dissous dans 200 ml d'acétate d'éthyle. On lave la solution deux fois avec 50 ml d'eau, on sèche sur sulfate de sodium et on concentre la solution. Le produit cristallise au cours de l'évaporation. On obtient 9,23 g de 2-[(4-morpholino-6-propyl-1,3,5-triazin-2-yl)amino]-éthanol identique au produit obtenu ci-dessus.

Exemple 3. Préparation selon le procédé (c).

a) Obtention des 2-amino-4-morpholino-6-propyl-1,3,5-triazines de départ.

1. 2-amino-4-morpholino-6-propyl-1,3,5-triazine (chlorhydrate).

Ce produit est décrit par S. MURAI et al. dans la demande de brevet japonaise 69.688/74 (Chem. Abstr. 81, (1974), 136188x). Il peut être préparé de la manière suivante.

On mélange 87 g (1 mole) de morpholine et 8,6 g (0,05 mole) de 2-amino-4-chloro-6-propyl-1,3,5-triazine. La température du mélange s'élève d'elle-même à 54°C. On chauffe ensuite à reflux pendant 5 heures. On évapore le mélange de réaction sous pression réduite et le résidu obtenu est redissous dans de l'acétate d'éthyle. On lave la solution trois fois à l'eau, puis on la sèche sur sulfate de sodium. Le solvant est éliminé sous pression réduite et le résidu est recristallisé dans de l'hexane. On obtient 9,1 g de 2-amino-4-morpholino-6-propyl-1,3,5-triazine.
Rendement : 82%. P.F. : 127-128°C.
Chlorhydrate : P.F. : 210-211°C (alcool isopropylique-éther)
Analyse pour $C_{10}H_{17}N_5O \cdot HCl$ en %

| | | | | |
|---|---|---|---|---|
| calculé: | C 46,24 | H 6,94 | N 26,97 | Cl- 13,68 |
| trouvé : | 46,21 | 6,90 | 26,78 | 13,61 |

La 2-amino-4-chloro-6-propyl-1,3,5-triazine utilisée comme produit de départ dans cette étape a été préparée selon la méthode de S. MURAI et al., (demande de brevet japonaise 69.688/74).

2. 2-(méthylamino)-4-morpholino-6-propyl-1,3,5-triazine (chlorhydrate).

A une solution contenant 5,6 g (0,03 mole) de 2-chloro-4-(méthylamino)-6-propyl-1,3,5-triazine dans 50 ml de dioxanne, on ajoute une solution contenant 8,7 g (0,1 mole) de morpholine dans 50 ml de dioxanne. Le mélange est chauffé à reflux pendant 5 heures. Ensuite, on refroidit et on filtre le chlorhydrate de morpholine qui s'est formé. Le solvant est éliminé sous pression réduite et le résidu est repris dans du chloroforme. On lave à l'eau et on sèche la phase organique sur sulfate de sodium. On évapore le solvant et le résidu est recristallisé dans l'acétate d'éthyle. On obtient 5,3 g de 2-(méthylamino)-4-morpholino-6-propyl-1,3,5-triazine.
Rendement : 74%. P.F. : 131-133°C.

Le produit obtenu est dissous dans de l'alcool isopropylique à chaud. A cette solution, on ajoute la quantité équivalente d'acide chlorhydrique en solution dans de l'éther éthylique. Le chlorhydrate cristallise par refroidissement. On filtre, on lave à l'éther éthylique et on sèche.
Rendement : 75%. P.F. : 177-178°C.
Analyse pour $C_{11}H_{19}N_5O \cdot HCl$ en %

| | | | | |
|---|---|---|---|---|
| calculé : | C 48,26 | H 7,31 | N 25,59 | Cl- 12,97 |
| trouvé : | 48,38 | 7,40 | 25,57 | 12,64 |

La 2-chloro-4-(méthylamino)-6-propyl-1,3,5-triazine utilisée comme produit de départ dans cette étape a été préparée selon la méthode de T. TSUJIKAWA et al., (Yakugaku Zasshi, 95, (1975), 512-520).

b) Préparation des 2-amino-4-morpholino-6-propyl-1,3,5-triazines.

1. N-(4-morpholino-6-propyl-1,3,5-triazin-2-yl)-acétamide (composé 21).

A la température ambiante, on ajoute goutte à goutte 4 g (0,05 mole) de chlorure d'acétyle dans une solution de 11,2 g (0,05 mole) de 2-amino-4-morpholino-6-propyl-1,3,5-triazine dans 100 ml de pyridine anhydre. On agite le mélange pendant 6 heures puis on laisse reposer pendant 48 heures. On filtre le chlorhydrate de pyridine et on évapore le filtrat sous pression réduite. Le résidu obtenu est repris une fois par du toluène que l'on évapore à nouveau. Ensuite, on reprend le résidu par du dichlorométhane, on lave la solution à l'eau et on sèche sur sulfate de sodium. Le résidu obtenu après évaporation du solvant est purifié par chromatographie sur silice (éluant : 90 : 10 (v/v) dichlorométhane-éthanol) et le produit est finalement recristallisé dans l'acétate d'éthyle. On obtient 5,75 g de N-(4-morpholino-6-propyl-1,3,5-triazin-2-yl)-acétamide.
Rendement : 43,4%. P.F. : 141-2°C. (Composé 21a)
Chlorhydrate : P.F. : 145-146°C (alcool isopropylique-éther).
(Composé 21b).
Analyse pour $C_{12}H_{19}N_5O_2 \cdot HCl$ en %

| | | | | |
|---|---|---|---|---|
| calculé : | C 47,76 | H 6,63 | N 23,22 | Cl- 11,77 |
| trouvé : | 47,78 | 6,73 | 22,80 | 11,62 |

2. N-(4-morpholino-6-propyl-1,3,5-triazin-2-yl)-benzamide (chlorhydrate) (composé 22).

Dans une solution de 11,2 g (0,05 mole) de 2-amino-4-morpholino-6-propyl-1,3,5-triazine dans 200 ml de dichloroéthane, on ajoute, à la température ambiante, successivement, une solution de 7,7 g (0,055 mole) de chlorure de benzoyle dans 50 ml de dichloroéthane et une solution de 5,5 g (0,055 mole) de triéthylamine dans 50 ml de dichloroéthane. On chauffe le mélange à reflux pendant 6 heures puis on le refroidit à la température

ambiante. On lave successivement à l'eau, avec une solution aqueuse de bicarbonate de sodium, puis encore une fois à l'eau. On sèche la phase organique sur sulfate de sodium et on évapore le solvant sous pression réduite. Le résidu obtenu est chromatographié sur silice (éluant : 95 : 5 (v/v) dichlorométhane-éthanol) puis finalement recristallisé dans un mélange 50 : 50 (v/v) éther-hexane. Le produit forme un chlorhydrate dans l'éther. On obtient ainsi 12,1 g de chlorhydrate de N-(4-morpholino-6-propyl-1,3,5-triazin-2-yl)-benzamide.
Rendement : 66,5%. P.F. : 197-198°C.
Analyse pour $C_{17}H_{21}N_5O_2 \cdot HCl$ en %

| | | | | |
|---|---|---|---|---|
| calculé : | C 56,12 | H 6,05 | N 19,26 | $Cl^-$ 9,77 |
| trouvé : | 56,30 | 6,50 | 19,16 | 9,54 |

Les composés réunis dans le tableau II ont été préparés, comme indiqué, soit selon la méthode de l'exemple 3b)1, soit selon la méthode de l'exemple 3b)2 ci-dessus.

## TABLEAU II

2-R-4-morpholino-6-propyl-1,3,5-triazines

| Composé | Substituant R | Méthode | Rdt (%) | P.F.(°C) | Analyses | calculé % | trouvé % |
|---|---|---|---|---|---|---|---|
| 23 | $NHCOCH_2C_6H_5$ | 3b.1 | 51,3 | 110-113 | C | 63,34 | 63,18 |
| | | | | | H | 6,74 | 6,85 |
| | | | | | N | 20,52 | 20,55 |
| 24 | $NHCOCH(CH_3)_2$ | 3b.1 | 34,3 | 141 | C | 57,34 | 56,84 |
| | | | | | H | 7,85 | 7,84 |
| | | | | | N | 23,89 | 23,70 |
| 25a | $N(CH_3)COCH_3$ | 3b.1 | 34,8 | 60-61 | C | 55,91 | 56,02 |
| | | | | | H | 7,53 | 7,57 |
| | | | | | N | 25,09 | 25,05 |
| 25b (1) | $N(CH_3)COCH_3$ | 3b.1 | 75,5 | 104-105 | C | 49,44 | 48,78 |
| | | | | | H | 6,97 | 7,01 |
| | | | | | N | 22,19 | 22,43 |
| 26 | $N(CH_3)COCH(CH_3)_2$ | 3b.1 | 38,5 | 36-37 | C | 58,62 | 58,78 |
| | | | | | H | 8,16 | 8,33 |
| | | | | | N | 22,80 | 22,62 |
| 27 (1) | $N(CH_3)COC_6H_5$ | 3b.2 | 46,5 | 122 | C | 57,22 | 56,78 |
| | | | | | H | 6,36 | 6,40 |
| | | | | | N | 18,54 | 18,28 |
| 28 | $NH-COOC_6H_5$ | 3b.2 | 24,8 | 154-155 | C | 59,48 | |
| | | | | | H | 6,12 | 6,32 |
| | | | | | N | 20,41 | 20,31 |
| 29 (1) | $NH-COC_6H_4-pCl$ | 3b.2 | 66 | 205-206 | C | 51,27 | 51,19 |
| | | | | | H | 5,31 | 5,34 |
| | | | | | N | 17,58 | 17,49 |

TABLEAU II (suite)

| Composé | Substituant R | Méthode | Rdt (%) | P.F. (°C) | Analyses | calculé % | trouvé % |
|---|---|---|---|---|---|---|---|
| 30 | $N(CH_3)COC_6H_4$-pCl | 3b.2 | 72 | 96-97 | C | 57,52 | 58,07 |
| | | | | | H | 5,90 | 6,17 |
| | | | | | N | 18,63 | 18,65 |
| 31 (1) | $NHCOC_6H_4$-p$OCH_3$ | 3b.2 | 33 | 199 | C | 54,89 | 54,83 |
| | | | | | H | 6,14 | 6,17 |
| | | | | | N | 17,78 | 17,80 |
| 32 (1) | $N(CH_3)COC_6H_4$-p$OCH_3$ | 3b.2 | 56 | 120 | C | 55,94 | 53,4 |
| | | | | | H | 6,42 | 6,48 |
| | | | | | N | 17,17 | 17,22 |
| 33 (1) | $NHCOC_6H_4$-p$CH_3$ | 3b.2 | 53 | 188 | C | 57,21 | 57,45 |
| | | | | | H | 6,40 | 6,42 |
| | | | | | N | 18,53 | 18,73 |
| 34 (1) | $N(CH_3)COC_6H_4$-p$CH_3$ | 3b.2 | 74 | 99 | C | 58,23 | 58 [illegible]3 |
| | | | | | H | 6,69 | 6,73 |
| | | | | | N | 17,87 | 18,11 |
| 35 | $N(CH_3)COC_6H_3$-(3,4-di-$OCH_3$) | 3b.2 | 70 | 73 | C | 59,84 | 60,12 |
| | | | | | H | 6,78 | 7,09 |
| | | | | | N | 17,44 | 17,45 |
| 36 | $NHCOC_6H_3$-(3,4-di-$CH_3$) | 3b.2 | 45 | 232 | C | 58,23 | 58,42 |
| | | | | | H | 6,69 | 6,65 |
| | | | | | N | 17,87 | 17,72 |

(1) chlorhydrate

Exemple 4. Préparation selon le procédé (d).

1. 2-[[2-(acétoxy)éthyl]amino]-4-morpholino-6-propyl-1,3,5-triazine (composé 37).

Dans une solution de 13,4 g (0,05 mole) de 2-[(4-morpholino-6-propyl-1,3,5-triazin-2-yl)amino]-éthanol (composé 1 préparé à l'exemple 1) dans 200 ml de dichlorométhane, on introduit simultanément, entre 0° et 5°C, 4,3 g (0,055 mole) de chlorure d'acétyle dissous dans 50 ml de dichlorométhane, et 5,5 g (0,055 mole) de triéthylamine en solution dans 50 ml de dichlorométhane. L'addition de ces deux réactifs est réglée de manière à ce que dans le milieu réactionnel, le chlorure d'acide soit toujours en excès par rapport à la triéthylamine. On maintient l'agitation du mélange pendant une heure à 5°C et ensuite pendant 12 heures à la température ambiante. On lave le mélange de réaction successivement avec une solution aqueuse de bicarbonate de sodium et avec de l'eau. On sèche sur sulfate de sodium et on évapore le solvant sous pression réduite. Le résidu cristallise dans un mélange toluène-hexane 1 : 2. Le produit obtenu est d'abord purifié par chromatographie sur silice (éluant : 95 : 5 (v/v) dichlorométhane-éthanol puis il est finalement recristallisé dans un mélange toluène-hexane 1 : 1. On obtient 9,3 g de 2-[[2-(acétoxy)éthyl]amino]-4-morpholino-6-propyl-1,3,5-triazine.
Rendement : 60%. P.F. : 94-95°C.
Analyse pour $C_{14}H_{23}N_5O_3$ en %

| | | | |
|---|---|---|---|
| calculé : | C 54,37 | H 7,44 | N 22,65 |
| trouvé : | 54,98 | 7,69 | 22,62 |

On prépare de la même manière les composés réunis dans le tableau III.

TABLEAU III

2-R-4-morpholino-6-propyl-1,3,5-triazines

| Composé | Substituant R | Rdt (%) | P.F. (°C) | Analyses | calculé % | trouvé % |
|---|---|---|---|---|---|---|
| 38 | $NH(CH_2)_2O-COCH(CH_3)_2$ | 59,3 | 54-55 | C | 56,97 | 56,86 |
| | | | | H | 8,01 | 8,04 |
| | | | | N | 20,77 | 20,74 |
| 39 | $NH(CH_2)_2O-COC_6H_5$ | 67,4 | 98-99 | C | 61,46 | 61,52 |
| | | | | H | 6,74 | 6,75 |
| | | | | N | 18,87 | 18,91 |
| 40 | $NH(CH_2)_2O-COCH_2C_6H_5$ | 63,4 | 63-64 | C | 62,34 | 62,45 |
| | | | | H | 7,01 | 7,07 |
| | | | | N | 18,18 | 18,15 |

Exemple 5. Préparation selon le procédé (e).

N,N-diméthyl-N'-(4-morpholino-6-propyl-1,3,5-triazin-2-yl)-urée (composé 41).

A la température de 20°C, on fait passer pendant trois heures un courant gazeux de diméthylamine dans une solution de 5 g (0,0145 mole) de (4-morpholino-6-propyl-1,3,5-triazin-2-yl)carbamate de phényle (composé 28 préparé à l'exemple 3) dans 100 ml de dichlorométhane anhydre. On laisse ensuite reposer le mélange pendant 12 heures. On le lave successivement avec une solution diluée d'hydroxyde de sodium et avec de l'eau. On sèche la phase organique sur sulfate de sodium et on évapore le solvant sous pression réduite. Le résidu obtenu cristallise dans un mélange 50 : 50 (v/v) acétate d'éthyle-hexane. On obtient 1,7 g de N,N-diméthyl-N'-(4-morpholino-6-propyl-1,3,5-triazin-2-yl)-urée.
Rendement : 39,9%. P.F. : 110-111°C.
Analyse pour $C_{13}H_{22}N_6O_2$ en %

| | | | |
|---|---|---|---|
| calculé : | C 53,06 | H 7,48 | N 28,57 |
| trouvé : | 53,20 | 7,69 | 28,67 |

Exemple 6. Préparation selon le procédé (f).

2-[[2-[(aminocarbonyl)oxy]éthyl]amino]-4-morpholino-6-propyl-1,3,5-triazine (composé 42).

Dans un litre d'ammoniac liquide, on introduit 19,4 g (0,05 mole) de 2-morpholino-4-[[2-[(phénoxycarbonyl)oxy]éthyl]amino]-6-propyl-1,3,5-triazine dissous dans 100 ml de dichlorométhane anhydre. On maintient l'agitation pendant 7 heures à une température comprise entre – 35°C et – 45°C. Ensuite, on évapore l'ammoniac et on lave la phase organique successivement avec une solution aqueuse de bicarbonate de sodium et avec de l'eau. On sèche sur sulfate de sodium et on évapore le solvant sous pression réduite. Le résidu obtenu cristallise dans un mélange 50 : 50 (v/v) acétate d'éthyle-hexane. On obtient 15,1 g de 2-[[2-[(aminocarbonyl)oxy]éthyl]amino]-4-morpholino-6-propyl-1,3,5-triazine.
Rendement : 97,4%. P.F. : 139-140°C.
Analyse pour $C_{13}H_{22}N_6O_3$ en %

| | | |
|---|---|---|
| calculé : | C 50,32 | H 7,09 |
| trouvé : | 50,39 | 7,15 |

La 2-morpholino-4-[[2-[(phénoxycarbonyl)oxy]éthyl]amino]-6-propyl-1,3,5-triazine utilisée comme produit de départ dans cet exemple, a été préparée selon le procédé de l'exemple 4 à partir de 2-[(4-morpholino-6-propyl-1,3,5-triazin-2-yl)amino]-éthanol et de chloroformiate de phényle.
Rendement 85%. P.F. : 83-84°C.
Analyse pour $C_{19}H_{25}N_5O_4$ en %

| | | | |
|---|---|---|---|
| calculé: | C 58,91 | H 6,46 | N 18,0 |
| trouvé : | 58,99 | 6,60 | 18,1 |

Exemple 7. Préparation selon le procédé (g).

N-[2-(acétoxy)éthyl]-N-(4-morpholino-6-propyl-1,3,5-triazin-2-yl)-acétamide (composé 43).

Dans une suspension de 13,4 g (0,05 mole) de 2-[(4-morpholino-6-propyl-1,3,5-triazin-2-yl)amino]-éthanol (composé 1 préparé à l'exemple 1) dans 250 ml de dichloroéthane, maintenue à une température voisine de 20°C, on ajoute une solution de 11,8 g (0,15 mole) de chlorure d'acétyle dans 50 ml de dichloroéthane et une solution de 15 g (0,15 mole) de triéthylamine dans 50 ml de dichloroéthane. Cette addition est effectuée de la manière suivante : on introduit d'abord la moitié du chlorure d'acide et on laisse réagir pendant 15 minutes, puis on introduit la moitié de la triéthylamine ; ensuite, on introduit le restant du chlorure d'acide, on laisse encore réagir pendant 15 minutes, on chauffe le mélange à 55°C et finalement on introduit le restant de la triéthylamine. Le mélange obtenu est chauffé à reflux pendant 7 heures. Puis, on le laisse reposer pendant une nuit à la température ordinaire. On le lave successivement avec une solution aqueuse de bicarbonate de sodium et avec de l'eau. On sèche sur sulfate de sodium et on filtre en présence de norite. Après évaporation du solvant sous pression réduite, le résidu obtenu est purifié par chromatographie sur silice (éluant : 95 : 5 (v/v) dichlorométhane-éthanol). Le produit obtenu cristallise à froid dans l'hexane. On obtient 6,35 g de N-[2-(acétoxy)éthyl]-N-(4-morpholino-6-propyl-1,3,5-triazin-2-yl)-acétamide.
Rendement : 36,2%. P.F. : 49-50°C.
Analyse pour $C_{16}H_{25}N_5O_4$ en %

| | | | |
|---|---|---|---|
| calculé : | C 54,70 | H 7,12 | N 19,94 |
| trouvé : | 55,22 | 7,22 | 20,0 |

Comme il a été indiqué plus haut, les 2-amino-4-morpholino-6-propyl-1,3,5-triazines de formule I, ainsi que leurs sels d'addition d'acides non toxiques pharmaceutiquement acceptables, possèdent la propriété de corriger les effets d'un hypofonctionnement du système cholinergique. Cette avantageuse propriété est mise en évidence par une série d'essais pharmacologiques par lesquels on montre que les composés conformes à l'invention présentent certains effets similaires à ceux de composés cholinergiques bien connus tels que l'oxotrémorine, l'arécoline ou la physostigmine, ou bien, s'opposent à l'effet d'un antagoniste cholinergique comme

la scopolamine.

Les composés de la présente invention ont été soumis à des essais pharmacologiques dont les résultats sont reproduits ci-après.

1. Potentialisation des effets cholinergiques de l'oxotrémorine.

Ce test (R.C. RATHBUN et al., Psychopharmacologia, 4, (1963), 114-125) a pour but de montrer que les composés conformes à l'invention potentialisent des effets cholinergiques centraux et périphériques provoqués par l'administration chez la souris d'une faible dose d'oxotrémorine.

Le degré d'activation cholinergique périphérique est mesuré par l'effet sialagogue quantifié par le système de cotation suivant :

- score 0 : la salive n'excède pas celle émise par une souris normale ;
- score 1 : un peu de salive se trouve autour des dents ;
- score 2 : la salive dessine une bande étroite autour de la bouche ;
- score 4 : la salive mouille une surface sous le menton ;
- score 6 : la salive coule de la bouche par exemple sur les membres antérieurs.

Les scores intermédiaires ne sont pas employés et la salive est enlevée après chaque observation.

Le degré d'activation cholinergique centrale est mesuré par l'effet trémogène quantifié par le système de cotation suivant :

- score 0 : pas de tremblement ;
- score 1 : tremblements occasionnels légers et périodiques ;
- score 2 : tremblements modérés et légers se répétant souvent ;
- score 4 : tremblements accentués, mais entrecoupés de périodes de calme ;
- score 6 : tremblements très accentués et quasi-continuels.

Des souris mâles NMRI (20 à 25 g) sont réparties en quatre groupes de cinq animaux, à savoir : un groupe témoin et trois groupes traités. Le composé à tester est administré par voie intrapéritonéale (aux trois groupes traités) 20 minutes avant l'administration de l'oxotrémorine, à une dose différente pour chacun des groupes traités. L'oxotrémorine est administrée par voie intrapéritonéale aux groupes traités et au groupe témoin, à la dose de 0,05 mg/kg, en solution dans 10 ml/kg de sérum physiologique. Cette dose correspond environ à la dose minimale d'oxotrémorine qui fait apparaître des tremblements et de la salivation.

Après l'administration de l'oxotrémorine, les animaux sont placés individuellement dans de petites cages et sont observés à intervalles réguliers de 5 minutes jusqu'à disparition complète des effets cholinergiques.

Pour chacun des groupes, les scores individuels relevés à chaque période d'observation sont additionnés; ceci permet d'établir une courbe représentant la somme des scores en fonction du temps, caractéristique de chaque groupe.

Les valeurs moyennes des surfaces sous les courbes obtenues pour chacun des groupes traités sont comparées à la valeur moyenne de la surface sous la courbe correspondant au groupe témoin et font l'objet d'une analyse statistique selon la méthode de Mann-Whitney. A partir de cette comparaison, on peut déterminer une "dose minimale active". Cette "dose minimale active" est la dose minimale de composé nécessaire pour observer encore une potentialisation de l'effet sialagogue ou trémogène de l'oxotrémorine, ou autrement dit, pour obtenir une surface plus grande que la surface sous la courbe obtenue pour le groupe témoin.

Les résultats obtenus à ce test ont montré que les composés de l'invention potentialisent l'effet sialagogue de l'oxotrémorine à une dose minimale comprise entre 7,7 et 107 mg/kg, et potentialisent l'effet trémogène à une dose minimale comprise entre 8 et 112 mg/kg. De plus, les doses minimales actives ne présentent pas d'effets cholinergiques propres et sont très éloignées des doses mortelles déterminées au test d'Irwin.

Par ailleurs, ce test a mis en évidence que certains composés conformes à l'invention, administrés à la dose minimale active, ont des effets de plus longue durée que l'ester méthylique de l'acide 1,2,5,6-tétrahydro-1-méthyl-3-pyridinecarboxylique (arécoline), composé cholinergique bien connu.

Ainsi, les composés de l'invention montrent une potentialisation des effets sialagogues et trémogènes de l'oxotrémorine vingt minutes après être administrés à la dose minimale active, alors que, dans les mêmes conditions, l'arécoline ne donne aucune potentialisation.

2. Inhibition de l'hyperactivité induite par la scopolamine.

Un animal placé pour la première fois dans un environnement nouveau manifeste une intense activité exploratrice de ce nouveau milieu. La diminution progressive et ensuite la disparition de cette activité exploratrice, en d'autres termes, l'habituation au nouveau milieu, peut être considérée comme une forme élémentaire d'apprentissage. Cette forme élémentaire d'apprentissage est sensible à l'influence des médicaments qui facilitent ou qui ralentissent l'apprentissage (A. PLATEL et al., Psychopharmacology, 78, (1982), 346-352). Ainsi par exemple, la scopolamine, composé qui provoque des troubles mnésiques, induit une hyperactivité exploratrice chez le rat placé dans un environnement nouveau ; ce phénomène est lié à l'activité anticholinergique centrale de ce composé (W.J. STEWART et al., Psychopharmacologia, 44, (1975), 291-295). Par contre, un agoniste cholinergique comme la physostigmine s'oppose à l'hyperactivité induite par la scopolamine.

Dans le test décrit ci-après, selon leur solubilité, les composés de l'invention sont administrés soit dans du sérum physiologique, soit dans un véhicule approprié (généralement un tampon citrate à pH 5). Ce test permet d'établir pour les composés conformes à l'invention une activité comparable à celle de la physostigmine.

Il est basé sur la technique originale décrite par A. PLATEL (loc. cit.), d'une part, et d'autre part sur le procédé d'automatisation de l'enregistrement des mesures décrit par H.J. TAUGER et al., (J.Neuroscience Methods, 10, (1984), 237-245). Dans ce test, on utilise des rats mâles Sprague-Dawley SPF (Specific Pathogen Free ; 160 à 200 g). Pendant la semaine qui précède l'expérience, ces animaux sont gardés dans des conditions normales par groupes de 15 animaux dans des cages grillagées standards, et la nourriture et la boisson sont librement accessibles.

Au début de l'expérience, les rats sont répartis en 4 groupes homogènes de 10 animaux et sont habitués pendant une heure au local d'expérimentation. Ensuite, chaque groupe d'animaux est soumis à un traitement déterminé :

— le groupe 1 reçoit deux injections intrapéritonéales simultanées de sérum physiologique ou du véhicule utilisé ;

— le groupe 2 reçoit une injection intrapéritonéale de sérum physiologique ou du véhicule utilisé et une injection intrapéritonéale de 0,5 mg/kg de scopolamine en solution ;

— le groupe 3 reçoit une injection intrapéritonéale du composé à tester en solution dans un véhicule approprié et une injection intrapéritonéale de sérum physiologique ou du véhicule utilisé ;

— le groupe 4 reçoit une injection intrapéritonéale du composé à tester et une injection intrapéritonéale de 0,5 mg/kg de scopolamine.

Trente minutes après ce traitement, les quatre groupes d'animaux sont testés simultanément. A cet effet, chaque groupe est réparti dans une enceinte carrée (100 × 100 cm) constituée d'un sol grillagé et d'une paroi verticale de 50 cm de hauteur. Chacune de ces enceintes contient 16 zones comprenant 4 zones dans les coins, 4 zones centrales et 8 zones périphériques. En outre, chaque enceinte est équipée de 2 rangées de cellules à infra-rouge disposées à 2 cm au-dessus du sol afin d'enregistrer les déplacements horizontaux des animaux et de 2 autres rangées de cellules à infra-rouge disposées à une hauteur de 10 cm du sol pour l'enregistrement des déplacements verticaux. Ces cellules à infra-rouge sont reliées à un microprocesseur qui permet la détermination de la moyenne des distances parcourues (en cm) par un groupe d'animaux, la détermination du nombre moyen de redressements effectués, de même que la répartition des déplacements horizontaux dans les différentes zones centrales, périphériques et des coins, exprimés par le temps moyen de séjour dans ces diverses zones. Chaque composé testé est étudié au moins à trois doses différentes à partir desquelles on détermine la dose minimale active qui inhibe l'hyperactivité induite par l'administration intrapéritonéale de 0,5 mg/kg de scopolamine. On compare ensuite soit les valeurs obtenues dans le groupe 2 traité seulement à la scopolamine avec celles qui sont obtenues dans le groupe 4 traité à la fois par la scopolamine et par le composé à tester, soit les valeurs obtenues dans le groupe 4 avec celles obtenues dans le groupe 3 traité seulement par le composé à tester. Le groupe témoin 1 sert de contrôle pour l'action de la scopolamine administrée au groupe 2. Les différences observées sont évaluées statistiquement dans tous les cas par la méthode de Mann-Whitney.

Les résultats obtenus dans ce test ont montré que les composés de l'invention possèdent une bonne activité inhibitrice de l'hyperactivité induite par la scopolamine. Les doses minimales actives déterminées pour ces composés sont comprises entre 1 et 89 mg/kg. Mais, contrairement à la physostigmine qui est inactive lorsqu'elle est administrée plus de 15 minutes avant d'effectuer les mesures, les composés conformes à l'invention possèdent encore une activité inhibitrice lorsqu'ils sont administrés 30 minutes avant d'effectuer les mesures. Leur activité est donc de plus longue durée que celle de la physostigmine.

3. Inhibition des effets de la scopolamine sur l'électroencéphalogramme (EEG).

L'administration de scopolamine à l'homme ou à l'animal induit des troubles mnésiques comparables à ceux qui apparaissent au cours du vieillissement normal ou pathologique. Chez des patients souffrant de démence sénile de type Alzheimer, on a obtenu une amélioration des troubles mnésiques par l'administration de physostigmine, composé qui inhibe l'acétyl-cholinestérase.

La scopolamine, administrée par voie intrapéritonéale à des rats, à la dose de 0,5 mg/kg, induit des variations du spectre EEG, qui se traduisent par une augmentation de la puissance de la bande de 8 Hz, une diminution de la puissance des bandes de 20,8 à 40 Hz ainsi qu'une augmentation de la puissance totale du spectre EEG. La physostigmine s'oppose à ces variations.

L'objectif de ce test est de montrer, au moyen de la méthode d'analyse quantitative de l'électroencéphalogramme, que les composés de l'invention ont la propriété de neutraliser les effets que la scopolamine exerce sur le spectre EEG (P. ETEVENON — L'Electroencéphalographie sur ordinateur. Analyse quantitative et statistique. Copedith, Paris, 1978).

Dans ce test, on utilise des rats mâles Albinos Sprague-Dawley SPF (160 à 200 g).

Lorsque les animaux sont âgés de 3 mois, on implante à demeure, aseptiquement et sous anesthésie générale, 5 électrodes corticales : une électrode indifférente, une électrode frontale gauche et une droite, et une électrode occipitale gauche et une droite (P. ETEVENON, loc. cit.).

Le test est effectué lorsque les animaux sont âgés d'environ 15 mois. Dans l'intervalle, les animaux sont maintenus dans des cages individuelles, ils reçoivent de l'eau et de la nourriture à volonté et subissent un cycle journalier régulier comportant une période d'obscurité entre 18 heures du soir et 6 heures du matin. En même temps, les animaux sont progressivement habitués aux cages de la cabine insonorisée qu'ils occuperont ultérieurement pour l'enregistrement des électroencéphalogrammes, de même qu'aux conditions expérimentales, par l'administration de placebo par voie intrapéritonéale.

Les produits sont administrés immédiatement avant l'enregistrement de l'EEG.

Les rats sont répartis par groupes de 8 animaux et on enregistre 16 échantillons de spectres EEG en 5 secondes (deux spectres EEG par animal). Les spectres obtenus sont ensuite analysés par ordinateur (transformée de Fourier rapide), ce qui permet de déterminer pour chaque groupe la moyenne des 16 mesures effectuées et d'en déduire, pour un animal, la puissance totale du spectre EEG ainsi que la distribution de cette puissance (en %) dans les différentes bandes de fréquences.

Cette opération (enregistrement des spectres) est répétée 9 fois (durée totale : 121 minutes).

L'effet du composé étudié est déduit de la comparaison statistique des résultats obtenus pour les différents groupes d'animaux auxquels ont été administrés respectivement le composé à tester, la scopolamine et un placébo.

Dans le tableau IV ci-dessous, on donne pour quelques composés de l'invention, administrés par voie intrapéritonéale à la dose indiquée en mg/kg, le pourcentage de réduction de l'augmentation de la puissance de la bande 6,4 à 9,6 Hz (moyenne 8 Hz), augmentation provoquée par l'administration intrapéritonéale de 0,5 mg/kg de scopolamine.

TABLEAU IV

Inhibition des effets de la scopolamine sur la bande de 6,4 à 9,6 Hz de l'EEG.

| Composé | Dose (mg/kg) | Inhibition (en %) |
|---|---|---|
| 1 | 5,3 | 52 |
| 7 | 29,5 | 50 |
| 9 | 8,8 | 53,3 |
| 20 | 2,7 | 66,7 |
| 21a | 2,7 | 100 |
| 22 | 3,6 | 66,7 |
| 25a | 0,88 | 46,7 |
| 26 | 3,1 | 55,6 |
| 27 | 12 | 93,8 |
| 37 | 9,9 | 53,3 |
| 43 | 11,2 | 77,8 |
| physostigmine | 0,4 | 76,8 |

Les résultats montrent que les composés de l'invention, comme la physostigmine, inhibent les effets que la scopolamine exerce sur l'électroencéphalogramme. On voit que cette inhibition atteint et dépasse même 50% à des doses relativement faibles, très éloignées des doses toxiques, ce qui n'est pas le cas pour la physostigmine.

4. Evitement passif à essais multiples.

Les composés de la présente invention ont été étudiés en vue de faire apparaître d'une part leur propriété de favoriser l'apprentissage, exprimée par la réduction du nombre d'essais nécessaires pour atteindre un critère prédéterminé, et d'autre part, leur propriété de s'opposer à l'amnésie provoquée par l'administration de scopolamine. A cet effet, on a utilisé la méthode de l'évitement passif à essais multiples. Cette méthode est bien connue pour évaluer les effets qu'un produit exerce sur la mémoire et l'apprentissage (A. FINE et al., Proc. Natl. Acad. Sci. USA, 82, (1985), 5227-5230).

Le test est pratiqué sur des rats mâles Sprague-Dawley (160-200 g) qui sont maintenus, pendant l'expérience, dans des cages standards. L'appareil utilisé est une cage transparente carrée, de 35 cm de côté et de 25 cm de hauteur, équipée d'un plancher grillagé électrifiable. Un tapis isolant (10 × 17 cm) en caoutchouc est placé sur le sol dans un des coins de la cage.

Pour évaluer si un composé est susceptible de favoriser l'apprentissage on effectue l'essai suivant.

Chaque animal est placé sur le tapis en caoutchouc et on note le temps que l'animal met pour se décider à quitter cet emplacement pour explorer la cage. Après 20 secondes d'exploration, l'animal reçoit un choc électrique (durée 3 secondes) dans les pattes, provoquant une réaction de fuite. Le rat est immédiatement retiré de l'appareil et replacé dans sa cage d'origine. On répète cette expérience jusqu'à ce que l'animal reste au moins 180 secondes sur le tapis de caoutchouc pour éviter le choc électrique. L'apprentissage est exprimé par le nombre moyen d'essais nécessaires pour atteindre un temps de séjour de 180 secondes sur le tapis.

Un temps de séjour de 180 secondes sur le tapis de caoutchouc est considéré comme étant la performance maximum à réaliser par l'animal pour éviter le choc électrique. Les rats qui séjournent pendant ce temps sur le tapis ont acquis le réflexe d'évitement et sont replacés dans leur cage d'origine sans recevoir de choc électrique. Pour évaluer si un composé est susceptible de favoriser la rétention mnésique au cours du temps on effectue l'expérience suivante. Chaque animal est soumis à quatre essais aux temps 0, 4, 24 et 28 heures. Au premier essai (temps 0), l'animal est placé sur le tapis en caoutchouc et on note le temps qu'il met pour se décider à quitter cet emplacement pour explorer la cage. Après 20 secondes d'exploration, le rat reçoit un choc

électrique (durée 3 secondes) dans les pattes, provoquant une réaction de fuite. Le rat est immédiatement retiré de l'appareil et replacé dans sa cage d'origine. Au cours des trois essais ultérieurs (temps : 4,24 et 28 heures), l'animal est replacé sur le tapis de caoutchouc et l'on note le temps mis pour quitter cet emplacement. Dès que les quatre pattes de l'animal reposent sur la grille, il reçoit un choc électrique et il est immédiatement retiré de l'appareil.

Au début de l'expérience, les rats sont répartis en 4 groupes homogènes de 15 animaux. Trente minutes avant chaque essai, chaque groupe d'animaux est soumis à un traitement déterminé :

— le groupe 1 reçoit une injection intrapéritonéale de sérum physiologique ;
— le groupe 2 reçoit une injection intrapéritonéale du composé à tester ;
— le groupe 3 reçoit une injection intrapéritonéale de 0,5 mg de scopolamine et
— le groupe 4 reçoit une injection intrapéritonéale de 0,5 mg de scopolamine et une injection intrapéritonéale du composé à tester, simultanément.

Les groupes 1 et 2 sont utilisés dans la première expérience et les groupes 3 et 4 dans la seconde expérience.

Les résultats obtenus dans ce test avec les composés de l'invention sont repris dans le tableau V. Dans ce tableau on indique le composé soumis à l'essai (colonne 1) et la dose administrée par voie intrapéritonéale, exprimée en mg/kg (colonne 2).

Dans les colonnes 3 et 4, on donne les résultats obtenus dans l'essai utilisé pour évaluer l'apprentissage. Les chiffres indiquent le nombre moyen d'essais nécessaires pour qu'un animal témoin (groupe 1) ou traité groupe 2) par le composé apprenne à séjourner pendant 180 secondes sur le tapis de caoutchouc pour éviter le choc électrique. Les résultats ont été analysés par le test de Student.

Dans les colonnes 5 à 12, on donne les résultats obtenus dans l'expérience utilisée pour évaluer la rétention mnésique. Dans les colonnes 5 à 8, les chiffres représentent les temps de séjour moyens observés respectivement aux temps 0, 4, 24 et 28 heures pour les animaux du groupe 3, traités seulement à la scopolamine, et dans les colonnes 9 à 12, on trouve les chiffres correspondants pour les animaux du groupe 4, traités simultanément par la scopolamine et par le composé étudié (à la dose indiquée dans la deuxième colonne). L'influence favorable d'un composé pour s'opposer à l'amnésie provoquée par la scopolamine est démontrée par l'augmentation du temps de séjour sur le tapis, à chaque observation. Les différences observées sont analysées statistiquement par la méthode de Mann-Whitney.

TABLEAU V

| Composé | Dose (mg/kg) | Apprentissage nombre moyen d'essais groupe 1 | Apprentissage nombre moyen d'essais groupe 2 | Rétention mnésique Temps de séjour (en secondes) groupe 3 0 | groupe 3 4 | groupe 3 24 | groupe 3 28 | groupe 4 0 | groupe 4 4 | groupe 4 24 | groupe 4 28 |
|---|---|---|---|---|---|---|---|---|---|---|---|
| 1 | 5,35 | 2,27 | 1,53 | 1,27 | 13,3 | 24,2 | 26,3 | 2,7 | 44,8 | 61,3 | 102,7 |
| 7 | 29,54 | 2,67 | 2,57 | 1,0 | 1,5 | 3,3 | 4,0 | 1,3 | 7,3 | 22,5 | 52,1 |
| 9 | 8,72 | 2,27 | 2,0 | 1,4 | 2,3 | 3,3 | 5,7 | 3,3 | 67,9 | 62,7 | 160,3 |
| 10 | 28,14 | 2,87 | 2,07 | 1,8 | 4,5 | 2,3 | 4,5 | 5,5 | 15,7 | 31,1 | 34,1 |
| 20 | 2,66 | 1,93 | 1,53 | 1,1 | 2,5 | 4,6 | 5,5 | 2,7 | 55,3 | 57,7 | 87,9 |
| 21a | 2,65 | 1,60 | 1,53 | 2,4 | 9,7 | 9,1 | 33,9 | 3,4 | 60,4 | 74,6 | 102,4 |
| 22 | 3,64 | 2,20 | 1,0 | 1,7 | 11,1 | 20,3 | 12,8 | 2,8 | 70,9 | 82,8 | 137,6 |
| 25a | 0,89 | 2,07 | 2,07 | 2,1 | 3,0 | 7,7 | 3,3 | 1,7 | 8,1 | 34,7 | 44,5 |
| 27 | 3,78 | 2,20 | 1,13 | 1,9 | 8,9 | 31,6 | 54,1 | 11,3 | 88,6 | 111,5 | 133,5 |
| 37 | 9,6 | 2,0 | 1,3 | 1,0 | 2,5 | 4,5 | 5,8 | 2,9 | 24,0 | 44,4 | 49,9 |
| 41 | 9,12 | 2,53 | 1,33 | 1,0 | 3,7 | 7,0 | 7,7 | 1,3 | 15,8 | 27,3 | 66,3 |
| physostigmine | 0,4 | 2,60 | 2,30 | 1,7 | 1,4 | 6,9 | 21,3 | 1,9 | 17,8 | 56,2 | 53,4 |

De ce tableau, il apparaît que :

— les composés de l'invention favorisent l'apprentissage du réflexe d'évitement : le nombre moyen d'essais nécessaires pour atteindre le critère prédéterminé (temps de séjour maximum de 180 secondes sur le tapis) est moins élevé pour les animaux traités (colonne 4) que pour les animaux témoins (colonne 3) ;

— l'effet amnésiant de la scopolamine est très marqué : on voit que les temps de séjour des animaux de groupe 3 (colonnes 5 à 8) sont nettement moindres que les 180 secondes réalisées par les témoins après un nombre d'essais (colonne 3) ; et, dans ces conditions, l'influence favorable des composés de l'invention pour s'opposer à l'effet amnésiant de la scopolamine est très nette : les animaux du groupe 4, traités simultanément par la scopolamine et par un composé de l'invention, ont des temps de séjour, à chaque observation, considérablement plus élevés que les animaux du groupe 3 traités à la scopolamine seule (comparer les résultats de la colonne 5 avec ceux de la colonne 9, 6 avec 10, etc.).

— la physostigmine exerce un effet favorable contre l'effet amnésiant de la scopolamine, similaire à celui des composés de l'invention mais à une dose présentant des effets secondaires et très proche de la dose toxique, ce qui n'est pas le cas pour les composés de l'invention.

5. Toxicité.

La toxicité des composés de l'invention a été déterminée chez la souris mâle NMRI au moyen du test d'Irwin (S. IRWIN, Psychopharmacologia, 13, (1968), 222-257).

Des doses progressives du composé à tester sont administrées par voie intrapéritonéale à des groupes de trois souris jusqu'à ce que la dose mortelle soit atteinte (dose provoquant la mort de deux animaux sur trois dans les 48 heures).

Dans le tableau VI ci-dessous, on donne la dose mortelle observée pour les composés de l'invention. Il ressort de ce tableau que les composés de l'invention sont très peu toxiques, contrairement à la physostigmine.

TABLEAU VI

| Composé | Dose mortelle (en mg/kg) | Composé | Dose mortelle (en mg/kg) |
|---|---|---|---|
| 1 | 802 | 23 | > 1024 |
| 2 | 718 | 24 | 880 |
| 3 | 844 | 25a | 838 |
| 4 | 886 | 25b | 947 |
| 5 | 563 | 26 | 922 |
| 6 | 886 | 27 | 1133 |
| 7 | 886 | 28 | > 1030 |
| 8 | 886 | 29 | 398 |
| 9 | 844 | 30 | 1125 |
| 10 | 844 | 31 | 511 |
| 11 | 892 | 32 | 1222 |
| 12 | 982 | 33 | 1132 |
| 13 | 934 | 34 | 1174 |
| 14 | > 1073 | 35 | 401 |
| 15 | 1121 | 36 | 1174 |
| 16 | 844 | 37 | 928 |
| 17 | 844 | 38 | > 1012 |
| 18 | 1007 | 39 | > 1114 |
| 19 | 1031 | 40 | 1157 |
| 20 | 799 | 41 | 294 |
| 21a | 996 | 42 | > 931 |
| 21b | 905 | 43 | 351 |
| 22 | 1091 | physostigmine | 0,82 |

6. Posologie et administration.

Les compositions pharmaceutiques renfermant les composés de l'invention peuvent être administrées par voie orale, parentérale ou rectale. Les compositions pharmaceutiques utilisables pour l'administration orale peuvent être solides ou liquides, par exemple sous forme de comprimés (enrobés on non), pilules, dragées, capsules en gélatine, solutions, sirops, etc. De même, les compositions utilisables pour l'administration par voie parentérale, sont les formes pharmaceutiquement connues pour ce genre d'administration, par exemple des solutions, des suspensions ou émulsions aqueuses ou huileuses.

Pour l'administration par voie rectale, les compositions contenant les composés de l'invention se présentent généralement sous forme de suppositoires.

Les formes pharmaceutiques telles que solutions injectables, suspensions injectables, comprimés, gouttes, suppositoires, etc. sont préparées selon les méthodes couramment utilisées par les pharmaciens. Les formes pharmaceutiques comprennent également les compositions qui permettent de délivrer le produit actif d'une manière progressive. On mélange les composés de l'invention avec un véhicule solide ou liquide, non toxique, pharmaceutiquement acceptable, et éventuellement avec un agent dispersant, un agent désintégrant, un agent stabilisant, etc. On peut y ajouter, le cas échéant, des agents édulcorants, des agents colorants, etc. Le pourcentage de produit actif dans les compositions pharmaceutiques peut varier dans des limites très larges,

selon le patient et le mode d'administration, en particulier selon la fréquence d'administration.

Quant à la posologie journalière, elle peut varier dans une gamme étendue de doses unitaires, par exemple de 0,1 à 2 g de produit actif, selon le mode d'administration. Ainsi, par exemple, elle peut être de 0,25 à 0,75 g, de préférence 0,5 g, une à trois fois par jour, lorsque le composé est administré sous forme de comprimé.

A titre d'exemple non limitatif d'une composition contenant un composé de formule I pouvant être administré par voie orale, on cite ci-après une formulation pour comprimés :

| | |
|---|---|
| composé 1 | 250 mg |
| méthylcellulose (Methocel K4M) | 200 mg |
| lactose sec | 154 mg |
| aérosil | 5 mg |
| acide citrique anhydre | 60 mg |
| talc | 11 mg |
| stéarate de magnesium | 20 mg |

## Revendications

### Revendications pour les Etats contractants suivants : AT, BE, CH, DE, FR, GB, IT, LI, LU, NL, SE

1. 2-Amino-4-morpholino-6-propyl-1,3,5-triazines, leurs isomères optiques et leurs mélanges racémiques, répondant à la formule générale

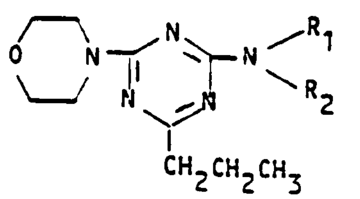

(I)

dans laquelle

$R_1$ représente un atome d'hydrogène, un radical alkyle, phénylalkyle ou acétyle,

$R_2$ représente un groupe hydroxyle, un radical hydroxalkyle, alkoxyalkyle, dialkylamino, phénylhydroxyalkyle, (hydroxycycloalkyl)alkyle, alkanoyloxyalkyle, benzoyloxyalkyle, phénylacétyloxyalkyle ou aminocarbonyloxyalkyle, un groupe $COR_3$ dans lequel $R_3$ représente un radical alkyle, phényle, halogénophényle, alkylphényle, alkoxyphényle, phénylalkyle ou phénoxy, ou un groupe $CONR_4R_5$ dans lequel $R_4$ et $R_5$ représentent de l'hydrogène ou un radical alkyle, ou

$R_1$ et $R_2$ forment conjointement avec l'atome d'azote auquel ils sont attachés un radical pyrrolidino ou pipéridino substitué par un radical hydroxyalkyle, les radicaux alkyle, alkoxy et alkanoyloxy ayant de 1 à 4 atomes de carbone et les radicaux cycloalkyle ayant de 4 à 6 atomes de carbone,

avec la restriction que lorsque $R_1$ représente le radical acétyle, $R_2$ représente un radical acétoxyalkyle, ainsi que leurs sels d'addition d'acides non toxiques pharmaceutiquement acceptables.

2. 2-[(4-morpholino-6-propyl-1,3,5-triazin-2-yl)amino]-éthanol et ses sels d'addition d'acides non toxiques pharmaceutiquement acceptables, selon la revendication 1.

3. 2-(hydroxyamino)-4-morpholino-6-propyl-1,3,5-triazine et ses sels d'addition d'acides non toxiques pharmaceutiquement acceptables, selon la revendication 1.

4. 2-[(2-méthoxyéthyl)amino]-4-morpholino-6-propyl-1,3,5-triazine et ses sels d'addition d'acides non toriques pharmaceutiquement acceptables, selon la revendication 1.

5. (S)-3-[(4-morpholino-6-propyl-1,3,5-triazin-2-yl)amino]-2-propanol et ses sels d'addition d'acides non

toxiques pharmaceutiquement acceptables, selon la revendication 1.

6. (R)-3-[(4-morpholino-6-propyl-1,3,5-triazin-2-yl)amino]-2-propanol et ses sels d'addition d'acides non toriques pharmaceutiquement acceptables, selon la revendication 1.

7. 2-(2,2-diméthylhydrazino)-4-morpholino-6-propyl-1,3,5-triazine et ses sels d'addition d'acides non toxiques pharmaceutiquement acceptables, selon la revendication 1.

8. N-(4-morpholino-6-propyl-1,3,5-triazin-2-yl)-benzamide et ses sels d'addition d'acides non toxiques pharmaceutiquement acceptables, selon la revendication 1.

9. N-méthyl-N-(4-morpholino-6-propyl-1,3,5-triazin-2-yl)-benzamide et ses sels d'addition d'acides non toriques pharmaceutiquement acceptables, selon la revendication 1.

10. 2-[[2-(acétoxy)éthyl]amino]-4-morpholino-6-propyl-1,3,5-triazine et ses sels d'addition d'acides non toxiques pharmaceutiquement acceptables, selon la revendication 1.

11. Procédé de préparation de 2-amino-4-morpholino-6-propyl-1,3,5-triazines et de leurs sels tels que définis dans la revendication 1, caractérisé en ce que

a) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un groupe hydroxyle, un radical hydroxyalkyle, alkoxyalkyle, dialkylamino, phényl-hydroxyalkyle ou (hydroxy-cycloalkyl)alkyle, ou $R_1$ et $R_2$ forment conjointement avec l'atome d'azote auquel ils sont attachés un radical pyrrolidino ou pipéridino substitué par un radical hydroxalkyle, les radicaux alkyle et alkoxy ayant de 1 à 4 atomes de carbone et les radicaux cycloalkyle ayant de 4 à 6 atomes de carbone, on fait réagir la 2-chloro-4-morpholino-6-propyl-1,3,5-triazine de formule

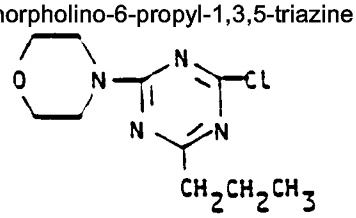

(II)

avec une amine de formule $HNR_1R_2$ (III) dans laquelle $R_1$ et $R_2$ ont la signification donnée ci-dessus, ou on fait réagir une 2-amino-4-chloro-6-propyl-1,3,5-triazine de formule

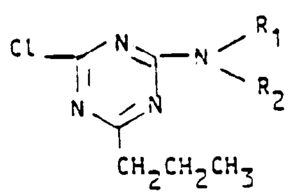

(IV)

dans laquelle $R_1$ et $R_2$ ont la signification donnée ci-dessus, avec une morpholine de formule

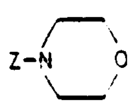

(V)

dans laquelle Z représente un atome d'hydrogène ou un radical méthyle ; ou

b) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un groupe $COR_3$ dans lequel $R_3$ représente un radical alkyle, phényle, halogénophényle, alkylphényle, alkoxyphényle, phénylalkyle ou phénoxy, les radicaux alkyle et alkoxy ayant de 1 à 4 atomes de carbone, on fait réagir, dans des proportions équimolaires, une 2-amino-4-morpholino-6-propyl-1,3,5-triazine de formule

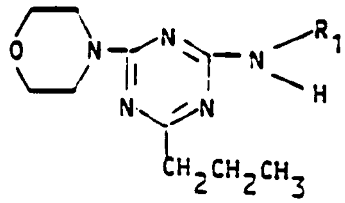

(VI)

dans laquelle $R_1$ a la signification donnée ci-dessus, avec un halogénure de $R_3$-carbonyle de formule $HalCOR_3$, (VII) dans laquelle $R_3$ a la signification donnée ci-dessus et Hal est un atome d'halogène ; ou

c) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un radical alkanoyloxyalkyle, benzoyloxyalkyle ou phénylacétyloxyalkyle, les radicaux alkyle et alkanoyle ayant de 1 à 4 atomes de carbone, on fait réagir, dans des proportions équimolaires, un (4-morpholino-6-propyl-1,3,5-triazin-2-yl)-aminoalcanol de formule

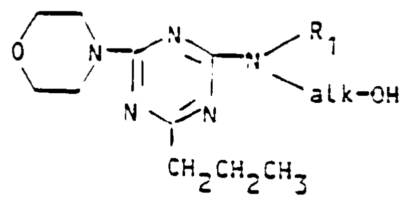

(VIII)

dans laquelle $R_1$ a la signification donnée ci-dessus et alk représente un radical alkylène ayant de 1 à 4 atomes de carbone, avec un halogénure de $R_6$-carbonyle de formule $HalCOR_6$ (IX) dans laquelle $R_6$ représente un radical alkyle ayant de 1 à 4 atomes de carbone, phényle ou benzyle et Hal est un atome d'halogène ; ou

d) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un groupe $CONR_4R_6$ dans lequel $R_4$ et $R_6$ représentent de l'hydrogène ou un radical alkyle, les radicaux alkyles ayant de 1 à 4 atomes de carbone, on fait réagir un (4-morpholino-6-propyl-1,3,5-triazin-2-yl)carbamate de phényle de formule

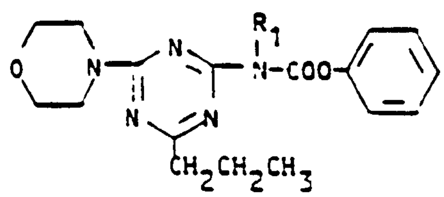

(X)

dans laquelle $R_1$ a la signification donnée ci-dessus, avec un composé azoté de formule $HNR_4R_6$ (XI) dans laquelle $R_4$ et $R_6$ ont la signification donnée ci-dessus ; ou

e) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un radical aminocarbonyloxyalkyle, les radicaux alkyles ayant de 1 à 4 atomes de carbone, on fait réagir de l'ammoniac arec une 2-morpholino-4-(phénoxycarbonyloxyalkylamino)-6-propyl-1,3,5-triazine de formule

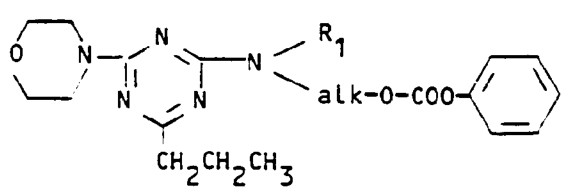

(XII)

dans laquelle $R_1$ a la signification donnée ci-dessus et alk représente un radical alkylène ayant de 1 à 4 atomes de carbone ; ou

f) pour préparer les 2-amino-4-morpholino-6-propyl-1,3,5-triazines répondant à la formule générale I dans laquelle $R_1$ représente le radical acétyle et $R_2$ un radical acétoxyalkyle dont le radical alkyle possède 1 à 4 atomes de carbone, on fait réagir au moins deux moles d'un halogénure d'acétyle avec une mole d'un (4-morpholino-6-propyl-1,3,5-triazin-2-yl)aminoalcanol de formule

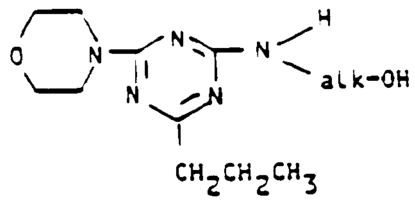

(XIV)

dans laquelle alk représente un radical alkylène ayant de 1 à 4 atomes de carbone ; et

g) en ce que, éventuellement, on convertit les 2-amino-4-morpholino-6-propyl-1,3,5-triazines de formule I ainsi obtenues en leurs sels d'addition d'acides non toxiques pharmaceutiquement acceptables.

12. 2-amino-4-morpholino-6-propyl-1,3,5-triazines selon l'une quelconque des revendications 1 à 10 ou un de leurs sel d'addition d'acides non toxiques pharmaceutiquement acceptables pour utilisation dans le traitement des troubles cognitifs et comportementaux associés à l'âge et aux syndromes démentiels.

13. Compositions thérapeutiques renfermant une quantité thérapeutiquement efficace d'une 2-amino-4-morpholino-6-propyl-1,3,5-triazine selon l'une quelconque des revendications 1 à 10 ou d'un de ses sels d'addition d'acides non toxiques, pharmaceutiquement acceptables, en association avec des excipients pharmaceutiques solides ou liquides.

**Revendication pour les Etats contractants suivants : ES, GR**

1. Procédé de préparation de 2-amino-4-morpholino-6-propyl-1,3,5-triazines, leurs isomères optiques et leurs mélanges racémiques, répondant à la formule générale

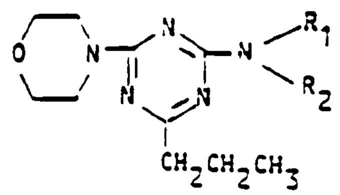

(I)

dans laquelle

$R_1$ représente un atome d'hydrogène, un radical alkyle, phénylalkyle ou acétyle,

$R_2$ représente un groupe hydroxyle, un radical hydroxyalkyle, alkoxyalkyle, dialkylamino, phénylhydroxyalkyle, (hydroxy-cycloalkyl)alkyle, alkanoyloxyalkyle, benzoyloxyalkyle, phénylacétyloxyalkyle ou aminocarbonyloxyalkyle, un groupe $COR_3$ dans lequel $R_3$ représente un radical alkyle, phényle, halogénophényle, alkylphényle, alkoxyphényle, phénylalkyle ou phénoxy, ou un groupe $CONR_4R_5$ dans lequel $R_4$ et $R_5$ représentent de l'hydrogène ou un radical alkyle, ou

$R_1$ et $R_2$ forment conjointement avec l'atome d'azote auquel ils sont attachés un radical pyrrolidino ou pipéridino substitué par un radical hydroxyalkyle, les radicaux alkyle, alkoxy et alkanoyloxy ayant de 1 à 4 atomes de carbone et les radicaux cycloalkyle ayant de 4 à 6 atomes de carbone,

avec la restriction que lorsque $R_1$ représente le radical acétyle, $R_2$ représente un radical acétoxyalkyle, ainsi que leurs sels d'addition d'acides non toxiques pharmaceutiquement acceptables, caractérisé en ce que:

a) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un groupe hydroxyle, un radical hydroxyalkyle, alkoxyalkyle, dialkylamino, phényl-hydroxyalkyle ou (hydroxy-cycloalkyl)alkyle, ou $R_1$ et $R_2$ forment conjointement avec l'atome d'azote auquel ils sont attachés un radical pyrrolidino ou pipéridino substitué par un radical hydroxyalkyle, les radicaux alkyle et alkoxy ayant de 1 à 4 atomes de carbone et les radicaux cycloalkyle ayant de 4 à 6 atomes de carbone, on fait réagir la 2-chloro-4-morpholino-6-propyl-1,3,5-triazine de formule

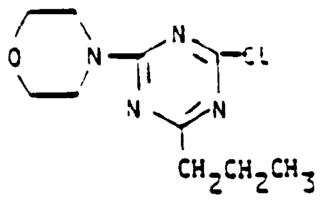

(II)

avec une amine de formule $HNR_1R_2$ (III) dans laquelle $R_1$ et $R_2$ ont la signification donnée ci-dessus, ou on fait réagir une 2-amino-4-chloro-6-1,3,5-triazine de formule

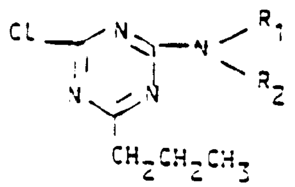

(IV)

dans laquelle $R_1$ et $R_2$ ont la signification donnée ci-dessus, avec une morpholine de formule

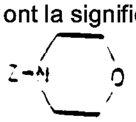

(V)

dans laquelle Z représente un atome d'hydrogène ou un radical méthyle ; ou

b) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un groupe $COR_3$ dans lequel $R_3$ représente un radical alkyle, phényle, halogénophényle, alkylphényle, alkoxyphényle, phénylalkyle ou phénoxy, les radicaux alkyle et alkoxy ayant de 1 à 4 atomes de carbone, on fait réagir, dans des proportions équimolaires, une 2-amino-4-morpholino-6-propyl-1,3,5-triazine de formule

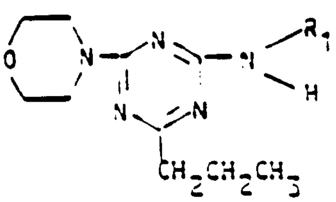

(VI)

dans laquelle $R_1$ a la signification donnée ci-dessus, avec un halogénure de $R_3$-carbonyle de formule $HalCOR_3$ (VII) dans laquelle $R_3$ a la signification donnée ci-dessus et Hal est un atome d'halogène ; ou

c) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un radical alkanoyloxyalkyle, benzoyloxyalkyle ou phénylacétyloxyalkyle, les radicaux alkyle et alkanoyle ayant de 1 à 4 atomes de carbone, on fait réagir, dans des proportions équimolaires, un (4-morpholino-6-propyl-1,3,5-triazin-2-yl)-aminoalcanol de formule

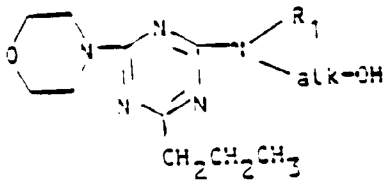

(VIII)

dans laquelle $R_1$ a la signification donnée ci-dessus et alk représente un radical alkylène ayant de 1 à 4 atomes de carbone, avec un halogénure de $R_6$-carbonyle de formule $HalCOR_6$ (IX) dans laquelle $R_6$ représente un radical alkyle ayant de 1 à 4 atomes de carbone, phényle ou benzyle et Hal est un atome d'halogène ; ou

d) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un

groupe $CONR_4R_5$ dans lequel $R_4$ et $R_5$ représentent de l'hydrogène ou un radical alkyle, les radicaux alkyles ayant de 1 à 4 atomes de carbone, on fait réagir un (4-morpholino-6-propyl-1,3,5-triazin-2-yl)carbamate de phényle de formule

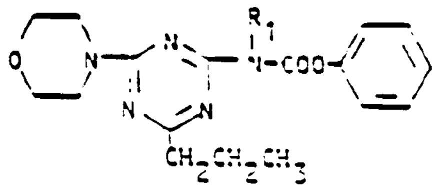

(X)

dans laquelle $R_1$ a la signification donnée ci-dessus, avec un composé azoté de formule $HNR_4R_5$ (XI) dans laquelle $R_4$ et $R_5$ ont la signification donnée ci-dessus ; ou

e) pour préparer les 2-amino-4-morpholino-6-propyl-1,3,5-triazines répondant à la formule générale I dans laquelle $R_1$ représente un atome d'hydrogène, un radical alkyle ou phénylalkyle et $R_2$ représente un radical aminocarbonyloxyalkyle, les radicaux alkyles ayant de 1 à 4 atomes de carbone, on fait réagir de l'ammoniac avec une 2-morpholino-4-(phénoxycarbonyloxyalkylamino)-6-propyl-1,3,5-triazine de formule

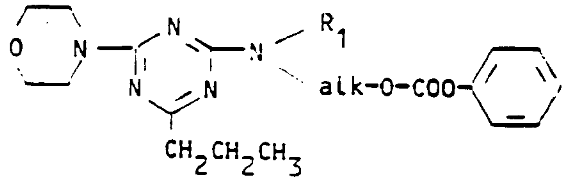

(XII)

dans laquelle $R_1$ a la signification donnée ci-dessus et alk représente un radical alkylène ayant de 1 à 4 atomes de carbone ; ou

f) pour préparer les 2-amino-4-morpholino-6-propyl-1,3,5-triazines répondant à la formule générale I dans laquelle $R_1$ représente le radical acétyle et $R_2$ un radical acétoxyalkyle dont le radical alkyle possède 1 à 4 atomes de carbone, on fait réagir au moins deux moles d'un halogénure d'acétyle avec une mole d'un (4-morpholino-6-propyl-1,3,5-triazin-2-yl)aminoalcanol de formule

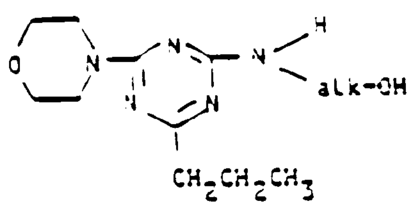

(XIV)

dans laquelle alk représente un radical alkylène ayant de 1 à 4 atomes de carbone ; et

g) en ce que, éventuellement, on convertit les 2-amino-4-morpholino-6-propyl-1,3,5-triazines de formule I ainsi obtenues en leurs sels d'addition d'acides non toxiques pharmaceutiquement acceptables.

## Patentansprüche

### Patentansprüche für folgende Vertragsstaaten : AT, BE, CH, DE, FR, GB, IT, LI, LU, NL, SE

1. 2-Amino-4-morpholino-6-propyl-1,3,5-triazine, deren optische Isomeren und racemischen Mischungen, der allgemeinen Formel

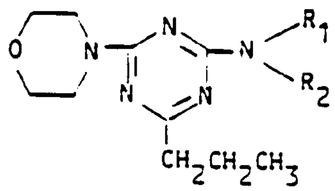

(I)

worin

$R_1$ ein Wasserstoffatom, einen Alkyl-, Phenylalkyl- oder Acetylrest darstellt,

$R_2$ eine Hydroxylgruppe, einen Hydroxyalkyl-, Alkoxyalkyl-, Dialkylamino-, Phenyl-hydroxyalkyl-, (Hydroxycycloalkyl)alkyl-, Alkanoyloxyalkyl-, Benzoyloxyalkyl-, Phenylacetyloxyalkyl- oder Aminocarbonyloxyalkylrest, eine Gruppe $COR_3$, in der $R_3$ einen Alkyl-, Phenyl-, Halogenphenyl-, Alkylphenyl-, Alkoxyphenyl-, Phenylalkyl- oder Phenoxyrest darstellt, oder eine Gruppe $CONR_4$ $R_5$, in der $R_4$ und $R_5$ Wasserstoff oder einen Alkylrest bedeuten, darstellt, oder

$R_1$ und $R_2$ zusammen mit dem Stickstoffatom, an das sie gebunden sind, einen durch einen Hydroxyalkylrest substituierten Pyrrolidino- oder Piperidinorest bilden, wobei die Alkyl- Alkoxy- und Alkanoyloxyreste 1 bis 4 Kohlenstoffatome und die Cycloalkylreste 4 bis 6 Kohlenstoffatome aufweisen,

mit der Massgabe, dass wenn $R_1$ den Acetylrest darstellt, $R_2$ einen Acetoxyalkylrest darstellt, sowie deren Additions-Salze nicht — toxischer, pharmazeutisch annehmbarer Säuren.

2. 2-[(4-Morpholino-6-propyl-1,3,5-triazin-2-yl)amino]-ethanol und dessen Additions-Salze nicht-toxischer, pharmazeutisch annehmbarer Säuren, nach Anspruch 1.

3. 2-(Hydroxyamino)-4-morpholino-6-propyl-1,3,5-triazin und dessen Additions-Salze nicht-toxischer, pharmazeutisch annehmbarer Säuren, nach Anspruch 1.

4. 2-[(2-Methoxyethyl)amino]-4-morpholino-6-propyl-1,3,5-triazin und dessen Additions-Salze nicht-toxischer, pharmazeutisch annehmbarer Säuren, nach Anspruch 1.

5. (S)-3-[(4-Morpholino-6-propyl-1,3,5-triazin-2-yl)amino]-2-propanol und dessen Additions-Salze nicht-toxischer, pharmazeutisch annehmbarer Säuren, nach Anspruch 1.

6. (R)-3-[(4-Morpholino-6-propyl-1,3,5-triazin-2-yl)amino]-2-propanol und dessen Additions-Salze nicht-toxischer, pharmazeutisch annehmbarer Säuren, nach Anspruch 1.

7. 2-(2,2-Dimethylhydrazino)-4-morpholino-6-propyl-1,3,5-triazin und dessen Additions-Salze nicht-toxischer, pharmazeutisch annehmbarer Säuren, nach Anspruch 1.

8. N-(4-Morpholino-6-propyl-1,3,5-triazin-2-yl)-benzamid und dessen Additions-Salze nicht-toxischer, pharmazeutisch annehmbarer Säuren, nach Anspruch 1.

9. N-Methyl-N-(4-morpholino-6-propyl-1,3,5-triazin-2-yl)-benzamid und dessen Additions-Salze nicht-toxischer, pharmazeutisch annehmbarer Säuren, nach Anspruch 1.

10. 2-[[2-(Acetoxy)ethyl]amino]-4-morpholino-6-propyl-1,3,5-triazin und dessen Additions-Salze nicht-toxischer, pharmazeutisch annehmbarer Säuren, nach Anspruch 1.

11. Verfahren zur Herstellung von 2-Amino-4-morpholino-6-propyl-1,3,5-triazinen und deren Salzen, wie in Anspruch 1 definiert, dadurch gekennzeichnet, daß man

a) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ eine Hydroxylgruppe, einen Hydroxyalkyl-, Alkoxyalkyl-, Dialkylamino-, Phenylhydroxyalkyl- oder (Hydroxycycloalkyl)alkylrest darstellt, oder $R_1$ und $R_2$ zusammen mit dem Stickstoffatom, an das sie gebunden sind, einen durch einen Hydroxyalkylrest substituierten Pyrrolidino- oder Piperidinorest bilden, wobei die Alkyl- und Alkoxyreste 1 bis 4 Kohlenstoffatome und die Cycloalkylreste 4 bis 6 Kohlenstoffatome aufweisen, das 2-Chloro-4-morpholino-6-propyl-1,3,5-triazin der Formel

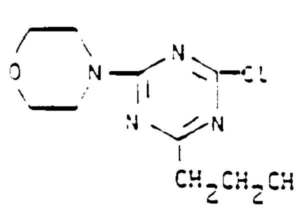

(II)

mit einem Amin der Formel $HNR_1 R_2$ (III) in der $R_1$ und $R_2$ die obengenannte Bedeutung aufweisen, umsetzt oder ein 2-Amino-4-chloro-6-propyl-1,3,5-triazin der Formel

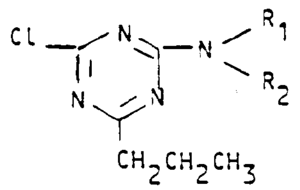

(IV)

in der $R_1$ und $R_2$ die obengenannte Bedeutung aufweisen, mit einem Morpholin der Formel

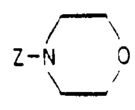

(V)

in der Z ein Wasserstoffatom oder einen Methylrest darstellt, umsetzt ; oder

b) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ eine Gruppe $COR_3$ darstellt, in der $R_3$ einen Alkyl-, Phenyl-, Halogenphenyl-, Alkylphenyl-, Alkoxyphenyl-, Phenylalkyl- oder Phenoxyrest darstellt, wobei die Alkyl- und Alkoxyreste 1 bis 4 Kohlenstoffatome aufweisen, in gleichmolaren Verhältnissen ein 2-Amino-4-morpholino-6-propyl-1,3,5-triazin der Formel

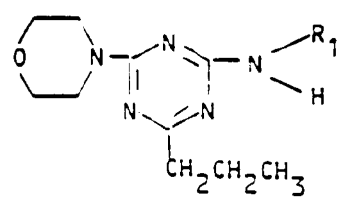

(VI)

in der $R_1$ die obengenannte Bedeutung aufweist, mit einem $R_3$-Carbonylhalogenid der Formel $HalCOR_3$ (VII), in der $R_3$ die obengenannte Bedeutung aufweist und Hal ein Halogenatom ist, umsetzt ; oder

c) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ einen Alkanoyloxyalkyl-, Benzoyloxyalkyl- oder Phenylacetyloxyalkylrest darstellt, wobei die Alkyl- und Alkanoylreste 1 bis 4 Kohlenstoffatome aufweisen, in gleichmolaren Verhältnissen ein (4-Morpholino-6-propyl-1,3,5-triazin-2-yl)-aminoalkanol der Formel

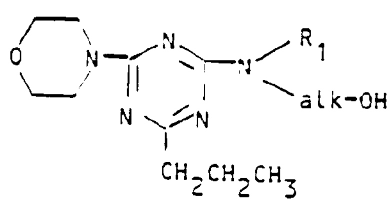

(VIII)

in der $R_1$ die obengenannte Bedeutung aufweist und alk einen Alkylenrest mit 1 bis 4 Kohlenstoffatomen darstellt, mit einem $R_6$-Carbonylhalogenid der Formel $HalCOR_6$ (IX), in der $R_6$ einen Alkylrest mit 1 bis 4 Kohlenstoffatomen, Phenyl- oder Benzylrest darstellt und Hal ein Halogenatom ist, umsetzt ; oder

d) zur Herstellung der 2-Amino-4-morpholino-6-propyl-1,3,5-triazine der allgemeinen Formel I, in der $R_1$ ein Wasserstoffatom, einen Alkyl- oder Phenylalkylrest darstellt, und $R_2$ eine Gruppe $CONR_4 R_6$ darstellt, in der $R_4$ und $R_5$ Wasserstoff oder einen Alkylrest darstellen, wobei die Alkylreste 1 bis 4 Kohlenstoffatome aufweisen, ein (4-Morpholino-6-propyl-1,3,5-triazin-2-yl)phenylcarbamat der Formel

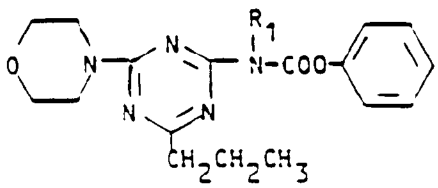

(X)

in der $R_1$ die obengenannte Bedeutung aufweist, mit einer stickstoffhaltigen Verbindung der Formel $HNR_4$ $R_5$ (XI), in der $R_4$ und $R_5$ die obengenannte Bedeutung aufweisen, umsetzt ; oder

e) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ einen Aminocarbonyloxyalkylrest darstellt, wobei die Alkylreste 1 bis 4 Kohlenstoffatome aufweisen, Ammoniak mit einem 2-Morpholino-4-(phenoxycarbonyloxyalkylamino)-6-propyl-1,3,5-triazin der Formel

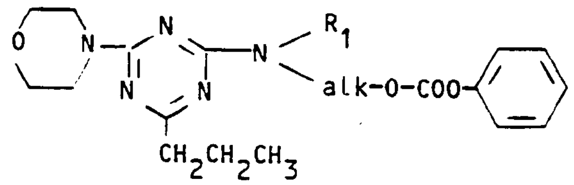

(XII)

in der $R_1$ die obengenannte Bedeutung aufweist, und alk einen Alkylenrest mit 1 bis 4 Kohlenstoffatomen darstellt, umsetzt ; oder

f) zur Herstellung der 2-Amino-4-morpholino-6-propyl-1,3,5-triazine der allgemeinen Formel I in der $R_1$ den Acetylrest und $R_2$ einen Acetoxyalkylrest, dessen Alkylrest 1 bis 4 Kohlenstoffatome aufweist, wenigstens 2 Mol eines Acetylhalogenids mit einem Mol eines (4-Morpholino-6-propyl-1,3,5-triazin-2-yl)aminoalkanols der Formel

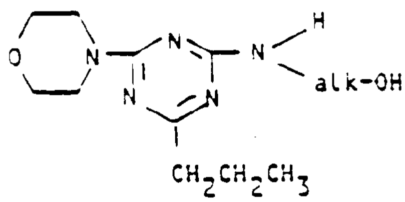

(XIV)

in der alk einen Alkylenrest mit 1 bis 4 Kohlenstoffatomen aufweist, umsetzt ; und

g) gegebenenfalls, die so erhaltenen 2-Amino-4-morpholino-6-propyl-1,3,5-triazine der Formel I in ihre Additions-Salze nichttoxischer, pharmazeutisch annehmbarer Säuren umwandelt.

12. 2-Amino-4-morpholino-6-propyl-1,3,5-triazine nach einem der Ansprüche 1 bis 10 oder eines ihrer Additions-Salze nicht-toxischer, pharmazeutisch annehmbarer Säuren zur Verwendung bei der Behandlung von Gedächtnis- und Verhaltensstörungen, die in Verbindung mit dem Alter und Demenz-Syndromen auftreten.

13. Heilmittel enthaltend eine therapeutisch wirksame Menge eines 2-Amino-4-morpholino-6-propyl-1,3,5-triazins nach einem der Ansprüche 1 bis 10 oder eines seiner Additions-Salze nicht-toxischer, pharmazeutisch annehmbarer Säuren in Verbindung mit festen oder flüssigen, pharmazeutischen Hilfstoffen.

**Patentanspruch für folgenden Vertragsstaaten : ES, GR**

1. Verfahren zur Herstellung von 2-Amino-4-morpholino-6-propyl-1,3,5-triazinen, deren optische Isomeren und racemischen Mischungen, der allgemeinen Formel

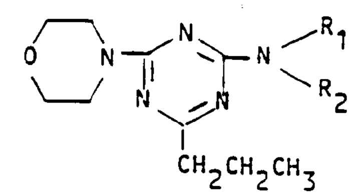

(I)

worin

- $R_1$ ein Wasserstoffatom, einen Alkyl-, Phenylalkyl- oder Acetylrest darstellt,
- $R_2$ eine Hydroxylgruppe, einen Hydroxyalkyl-, Alkoxyalkyl-, Dialkylamino-, Phenyl-hydroxyalkyl-, (Hydroxycycloalkyl)alkyl-, Alkanoyloxyalkyl-, Benzoyloxyalkyl-, Phenylacetyloxyalkyl- oder Aminocarbonyloxyalkylrest, eine Gruppe $COR_3$, in der $R_3$ einen Alkyl-, Phenyl-, Halogenphenyl-, Alkylphenyl-, Alkoxyphenyl-, Phenylalkyl- oder Phenoxyrest darstellt, oder eine Gruppe $CONR_4$ $R_5$, in der $R_4$ und $R_5$ Wasserstoff oder ein Alkylrest bedeuten, darstellt, oder
- $R_1$ und $R_2$ zusammen mit dem Stickstoffatom, an das sie gebunden sind, einen durch einen Hydroxyalkylrest substituierten Pyrrolidino- oder Piperidinorest bilden, wobei die Alkyl- Alkoxy- und Alkanoyloxyreste 1 bis 4 Kohlenstoffatome und die Cycloalkylreste 4 bis 6 Kohlenstoffatome aufweisen,

mit der Massgabe, dass wenn $R_1$ den Acetylrest darstellt, $R_2$ einen Acetoxyalkylrest darstellt, sowie deren Additions-Salze nicht — toxischer, pharmazeutisch annehmbarer Säuren, dadurch gekennzeichnet dass man

a) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ eine Hydroxylgruppe, einen Hydroxyalkyl-, Alkoxyalkyl-, Dialkylamino-, Phenylhydroxyalkyl- oder (Hydroxycycloalkyl)alkylrest darstellt, oder $R_1$ und $R_2$ zusammen mit dem Stickstoffatom, an das sie gebunden sind, einen durch einen Hydroxyalkylrest substituierten Pyrrolidino- oder Piperidinorest bilden, wobei die Alkyl- und Alkoxyreste 1 bis 4 Kohlenstoffatome und die Cycloalkylreste 4 bis 6 Kohlenstoffatome aufweisen, das 2-Chloro-4-morpholino-6-propyl-1,3,5-triazin der Formel

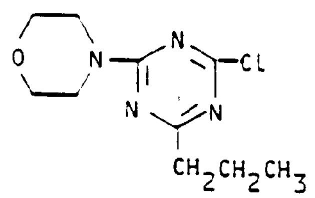

(II)

mit einem Amin der Formel $HNR_1$ $R_2$ (III) in der $R_1$ und $R_2$ die obengenannte Bedeutung aufweisen, umsetzt oder ein 2-Amino-4-chloro-6-propyl-1,3,5-triazin der Formel

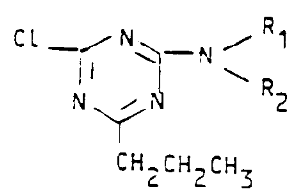

(IV)

in der $R_1$ und $R_2$ die obengenannte Bedeutung aufweisen, mit einem Morpholin der Formel

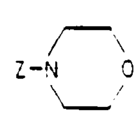

(V)

in der Z ein Wasserstoffatom oder einen Methylrest darstellt, umsetzt ; oder

b) zur Herstellung der 2-Amino-4-morpholino-6-propyl-1,3,5-triazine der allgemeinen Formel I in der $R_1$ ein

Wasserstoffatom, einen Alkyl- oder Phenylalkylrest darstellt und $R_2$ eine Gruppe $COR_3$ darstellt, in der $R_3$ einen Alkyl-, Phenyl-, Halogenphenyl-, Alkylphenyl-, Alkoxyphenyl-, Phenylalkyl- oder Phenoxyrest darstellt, wobei die Alkyl- und Alkoxyreste 1 bis 4 Kohlenstoffatome aufweisen, in gleichmolaren Verhältnissen ein 2-Amino-4-morpholino-6-propyl-1,3,5-triazin der Formel

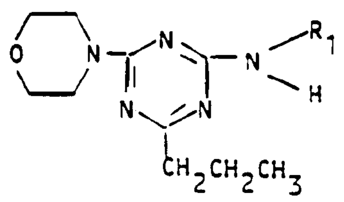 (VI)

in der $R_1$ die obengenannte Bedeutung aufweist, mit einem $R_3$-Carbonylhalogenid der Formel $HalCOR_3$ (VII), in der $R_3$ die obengenannte Bedeutung aufweist und Hal ein Halogenatom ist, umsetzt ; oder

c) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ einen Alkanoyloxyalkyl-, Benzoyloxyalkyl- oder Phenylacetyloxyalkylrest darstellt, wobei die Alkyl- und Alkanoylreste 1 bis 4 Kohlenstoffatome aufweisen, in gleichmolaren Verhältnissen ein (4-Morpholino-6-propyl-1,3,5-triazin-2-yl)-aminoalkanol der Formel

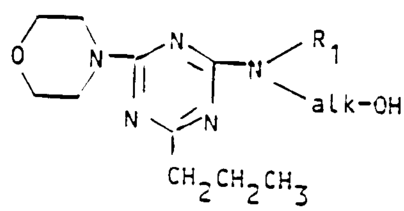 (VIII)

in der $R_1$ die obengenannte Bedeutung aufweist und alk einen Alkylenrest mit 1 bis 4 Kohlenstoffatomen darstellt, mit einem $R_6$-Carbonylhalogenid der Formel $HalCOR_6$ (IX), in der $R_6$ einen Alkylrest mit 1 bis 4 Kohlenstoffatomen, Phenyl- oder Benzylrest darstellt und Hal ein Halogenatom ist, umsetzt ; oder

d) zur Herstellung der 2-Amino-4-morpholino-6-propyl-1,3,5-triazine der allgemeinen Formel I, in der $R_1$ ein Wasserstoffatom, einen Alkyl- oder Phenylalkylrest darstellt, und $R_2$ eine Gruppe $CONR_4$ $R_6$ darstellt, in der $R_4$ und $R_5$ Wasserstoff oder einen Alkylrest darstellen, wobei die Alkylreste 1 bis 4 Kohlenstoffatome aufweisen, ein (4-Morpholino-6-propyl-1,3,5-triazin-2-yl)phenylcarbamat der Formel

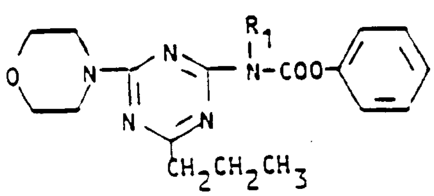 (X)

in der $R_1$ die obengenannte Bedeutung aufweist, mit einer stickstoffhaltigen Verbindung der Formel $HNR_4$ $R_5$ (XI), in der $R_4$ und $R_6$ die obengenannte Bedeutung aufweisen, umsetzt ; oder

e) zur Herstellung der 2-Amino-4-morpholino-6-propyl-1,3,5-triazine der allgemeinen Formel I in der $R_1$ ein Wasserstoffatom, einen Alkyl- oder Phenylalkylrest darstellt und $R_2$ einen Aminocarbonyloxyalkylrest darstellt, wobei die Alkylreste 1 bis 4 Kohlenstoffatome aufweisen, Ammoniak mit einem 2-Morpholino-4-(phenoxycarbonyloxyalkylamino)-6-propyl-1,3,5-triazin der Formel

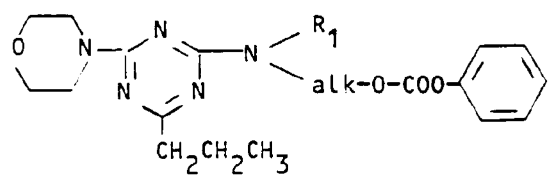 (XII)

in der $R_1$ die obengenannte Bedeutung aufweist, und alk einen Alkylenrest mit 1 bis 4 Kohlenstoffatomen darstellt, umsetzt ; oder

f) zur Herstellung der 2-Amino-4-morpholino-6-propyl-1,3,5-triazine der allgemeinen Formel I in der $R_1$ den Acetylrest und $R_2$ einen Acetoxyalkylrest, dessen Alkylrest 1 bis 4 Kohlenstoffatome aufweist, wenigstens 2 Mol eines Acetylhalogenids mit einem Mol eines (4-Morpholino-6-propyl-1,3,5-triazin-2-yl)aminoalkanols der Formel

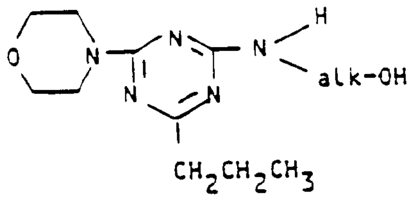

(XIV)

in der alk einen Alkylenrest mit 1 bis 4 Kohlenstoffatomen aufweist, umsetzt ; und

g) gegebenenfalls, die so erhaltenen 2-Amino-4-morpholino-6-propyl-1,3,5-triazine der Formel I in ihre Additions-Salze nichttoxischer, pharmazeutisch annehmbarer Säuren umwandelt.

## Claims

### Claims for the following Contracting States : AT, BE, CH, DE, FR, GB, IT, LI, LU, NL, SE

1. 2-amino-4-morpholino-6-propyl-1,3,5-triazines, their optical isomers and their racemic mixtures having the general formula

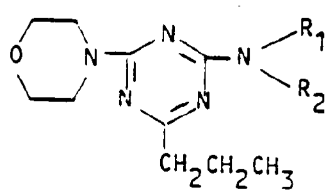

(I)

wherein

$R_1$ represents a hydrogen atom, an alkyl, phenylalkyl or acetyl radical,

$R_2$ represents a hydroxyl group, a hydroxyalkyl, alkoxyalkyl, dialkylamino, phenyl-hydroxyalkyl, (hydroxy-cycloalkyl)alkyl, alkanoyloxyalkyl, benzoyloxyalkyl, phenylacetyloxyalkyl or aminocarbonyloxyalkyl radical, a $COR_3$ group in which $R_3$ represents an alkyl, phenyl, halophenyl, alkylphenyl, alkoxyphenyl, phenylalkyl or phenoxy radical, or a $CONR_4$ $R_5$ group in which $R_4$ and $R_5$ represent hydrogen or an alkyl radical, or

$R_1$ and $R_2$ together with the nitrogen atom to which they are attached form a pyrrolidino or piperidino radical substituted by a hydroxyalkyl radical, the alkyl, alkoxy and alkanoyloxy radicals having 1 to 4 carbon atoms and the cycloalkyl radicals having 4 to 6 carbon atoms,

with the proviso that when $R_1$ represents the acetyl radical, $R_2$ represents an acetoxyalkyl radical, as well as their non-toxic pharmaceutically acceptable acid addition salts.

2. 2-[(4-morpholino-6-propyl-1,3,5-triazin-2-yl)amino]-ethanol and its non-toxic pharmaceutically acceptable acid addition salts, according to claim 1.

3. 2-(hydroxyamino)-4-morpholino-6-propyl-1,3,5-triazine and its non-toxic pharmaceutically acceptable acid addition salts, according to claim 1.

4. 2-[(2-methoxyethyl)amino]-4-morpholino-6-propyl-1,3,5-triazine and its non-toxic pharmaceutically acceptable acid addition salts, according to claim 1.

5. (S)-3-[(4-morpholino-6-propyl-1,3,5-triazin-2-yl)amino]-2-propanol and its non-toxic pharmaceutically acceptable acid addition salts, according to claim 1.

6. (R)-3-[(4-morpholino-6-propyl-1,3,5-triazin-2-yl)amino]-2-propanol and its non-toxic pharmaceutically acceptable acid addition salts, according to claim 1.

7. 2-(2,2-dimethylhydrazino)-4-morpholino-6-propyl-1,3,5-triazine and its non-toxic pharmaceutically acceptable acid addition salts, according to claim 1.

8. N-(4-morpholino-6-propyl-1,3,5-triazin-2-yl)-benzamide and its non-toxic pharmaceutically acceptable acid addition salts, according to claim 1.

9. N-methyl-N-(4-morpholino-6-propyl-1,3,5-triazin-2-yl)-benzamide and its non-toxic pharmaceutically acceptable acid addition salts, according to claim 1.

10. 2-[[2-(acetoxy)ethyl]amino]-4-morpholino-6-propyl-1,3,5-triazine and its non-toxic pharmaceutically acceptable acid addition salts, according to claim 1.

11. Process for the preparation of 2-amino-4-morpholino-6-propyl-1,3,5-triazines and their salts as defined in claim 1, characterised in that

a) in order to prepare the 2-amino-4-morpholino-6-propyl-1,3,5-triazines having the general formula I wherein $R_1$ represents a hydrogen atom, an alkyl or phenylalkyl radical and $R_2$ represents a hydroxyl group, a hydroxyalkyl, alkoxyalkyl, dialkylamino, phenylhydroxyalkyl or (hydroxy-cycloalkyl)alkyl radical, or $R_1$ and $R_2$ together with the nitrogen atom to which they are attached, form a pyrrolidino or piperidino radical substituted by a hydroxyalkyl radical, the alkyl and alkoxy radicals having 1 to 4 carbon atoms and the cycloalkyl radicals having 4 to 6 carbon atoms, 2-chloro-4-morpholino-6-propyl-1,3,5-triazine of the formula

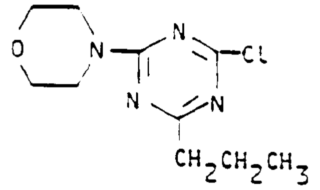

(II)

is reacted with an amine of the formula $HNR_1R_2$ (III) in which $R_1$ and $R_2$ have the meaning given above, or a 2-amino-4-chloro-6-propyl-1,3,5-triazine of the formula

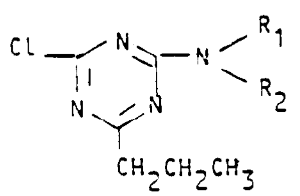

(IV)

in which $R_1$ and $R_2$ have the meaning given above, is reacted with a morpholine of the formula

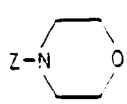

(V)

in which Z represents a hydrogen atom or a methyl radical ; or

b) in order to prepare the 2-amino-4-morpholino-6-propyl-1,3,5-triazines having the general formula I wherein $R_1$ represents a hydrogen atom, an alkyl or phenylalkyl radical and $R_2$ represents a $COR_3$ group in which $R_3$ is an alkyl, phenyl, halophenyl, alkylphenyl, alkoxyphenyl, phenylalkyl or phenoxy radical, the alkyl and alkoxy radicals having 1 to 4 carbon atoms, a 2-amino-4-morpholino-6-propyl-1,3,5-triazine of the formula

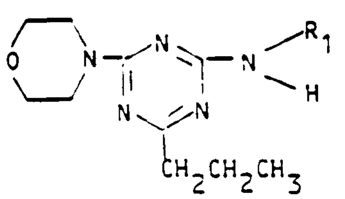

(VI)

in which $R_1$ has the meaning given above, is reacted in equimolar proportions with an $R_3$-carbonyl halide of the formula $HalCOR_3$ (VII) in which $R_3$ has the meaning given above and Hal is a halogen atom ; or

c) in order to prepare the 2-amino-4-morpholino-6-propyl-1,3,5-triazines having the general formula I wherein $R_1$ represents a hydrogen atom, an alkyl or phenylalkyl radical and $R_2$ represents an alkanoyloxyal-

kyl, benzoyloxyalkyl or phenylacetyloxyalkyl radical, the alkyl and alkanoyl radicals having 1 to 4 carbon atoms, a (4-morpholino-6-propyl-1,3,5-triazin-2-yl)-aminoalkanol of the formula

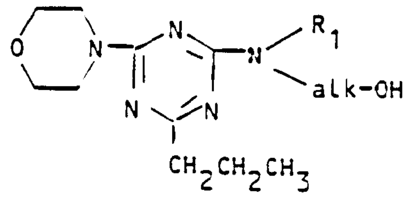

(VIII)

in which $R_1$ has the meaning given above and alk represents an alkylene radical having 1 to 4 carbon atoms, is reacted in equimolar proportions with an $R_6$-carbonyl halide of the formula $HalCOR_6$ (IX) in which $R_6$ represents an alkyl radical having 1 to 4 carbon atoms, phenyl or benzyl and Hal is a halogen atom ; or

d) in order to prepare the 2-amino-4-morpholino-6-propyl-1,3,5-triazines having the general formula I wherein $R_1$ represents a hydrogen atom, an alkyl or phenylalkyl radical and $R_2$ represents a $CONR_4 R_5$ group, in which $R_4$ and $R_5$ represent hydrogen or an alkyl radical, the alkyl radicals having 1 to 4 carbon atoms, a phenyl (4-morpholino-6-propyl-1,3,5-triazin-2-yl)carbamate of the formula

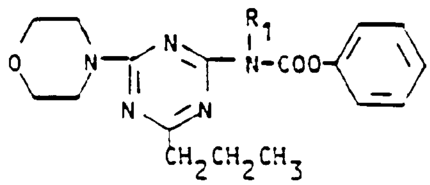

(X)

in which $R_1$ has the meaning given above, is reacted with a nitrogen compound of the formula $HNR_4 R_6$ (XI) in which $R_4$ and $R_6$ have the meaning given above ; or

e) in order to prepare the 2-amino-4-morpholino-6-propyl-1,3,5-triazines having the general formula I wherein $R_1$ represents a hydrogen atom, an alkyl or phenylalkyl radical and $R_2$ represents an aminocarbonyloxyalkyl radical, the alkyl radicals having 1 to 4 carbon atoms, ammonia is reacted with a 2-morpholino-4-(phenoxycarbonyloxyalkylamino)-6-propyl-1,3,5-triazine of the formula

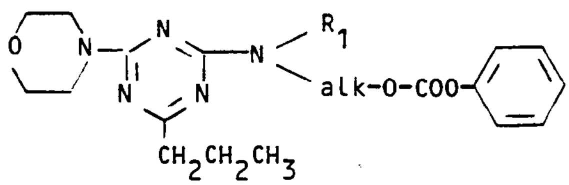

(XII)

in which $R_1$ has the meaning given above and alk represents an alkylene radical having 1 to carbon atoms ; or

f) in order to prepare the 2-amino-4-morpholino-6-propyl-1,3,5-triazines having the general formula I wherein $R_1$ represents the acetyl radical and $R_2$ an acetoxyalkyl radical, the alkyl radical of which has 1 to 4 carbon atoms, at least two moles of an acetyl halide are reacted with one mole of a (4-morpholino-6-propyl-1,3,5-triazin-2-yl)aminoalkanol of the formula

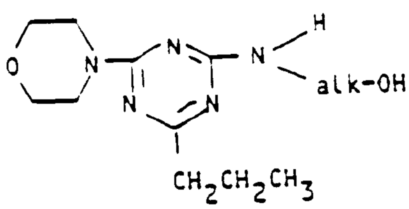

(XIV)

in which alk represents an alkylene radical having 1 to 4 carbon atoms ; and

g) optionally, the 2-amino-4-morpholino-6-propyl-1,3,5-triazines of the formula I thus obtained are converted into their non-toxic pharmaceutically acceptable acid addition salts.

12. 2-amino-4-morpholino-6-propyl-1,3,5-triazines according to any of claims 1 to 10 or one of their non-toxic pharmaceutically acceptable acid addition salts for use in the treatment of cognitive and behavioural disorders associated with aging and with dementia syndromes.

13. Therapeutic compositions containing a therapeutically effective amount of a 2-amino-4-morpholino-6-propyl-1,3,5-triazine according to any of claims 1 to 10 or one of its non-toxic pharmaceutically acceptable acid addition salts in association with solid or liquid pharmaceutical excipients.

**Claim for the following Contracting States : ES, GR**

1. Process for the preparation of 2-amino-4-morpholino-6-propyl-1,3,5-triazines, their optical isomers and their racemic mixtures having the general formula

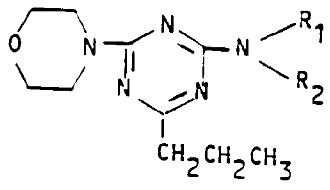

(I)

wherein

- $R_1$ represents a hydrogen atom, an alkyl, phenylalkyl or acetyl radical,
- $R_2$ represents a hydroxyl group, a hydroxyalkyl, alkoxyalkyl, dialkylamino, phenyl-hydroxyalkyl, (hydroxy-cycloalkyl)alkyl, alkanoyloxyalkyl, benzoyloxyalkyl, phenylacetyloxyalkyl or aminocarbonyloxyalkyl radical, a $COR_3$ group in which $R_3$ represents an alkyl, phenyl, halophenyl, alkylphenyl, alkoxyphenyl, phenylalkyl or phenoxy radical, or a $CONR_4R_5$ group in which $R_4$ and $R_5$ represent hydrogen or an alkyl radical, or
- $R_1$ and $R_2$ together with the nitrogen atom to which they are attached form a pyrrolidino or piperidino radical substituted by a hydroxyalkyl radical, the alkyl, alkoxy and alkanoyloxy radicals having 1 to 4 carbon atoms and the cycloalkyl radicals having 4 to 6 carbon atoms,

with the proviso that when $R_1$ represents the acetyl radical, $R_2$ represents an acetoxyalkyl radical, as well as their non-toxic pharmaceutically acceptable acid addition salts characterised in that :

a) in order to prepare the 2-amino-4-morpholino-6-propyl-1,3,5-triazines having the general formula I wherein $R_1$ represents a hydrogen atom, an alkyl or phenylalkyl radical and $R_2$ represents a hydroxyl group, a hydroxyalkyl, alkoxyalkyl, dialkylamino, phenyl-hydroxyalkyl or (hydroxy-cycloalkyl)alkyl radical, or $R_1$ and $R_2$ together with the nitrogen atom to which they are attached, form a pyrrolidino or piperidino radical substituted by a hydroxyalkyl radical, the alkyl and alkoxy radicals having 1 to 4 carbon atoms and the cycloalkyl radicals having 4 to 6 carbon atoms, 2-chloro-4-morpholino-6-propyl-1,3,5-triazine of the formula

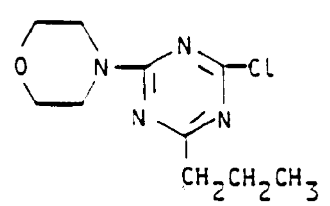

(II)

is reacted with an amine of the formula $HNR_1R_2$ (III) in which $R_1$ and $R_2$ have the meaning given above, or a 2-amino-4-chloro-6-propyl-1,3,5-triazine of the formula

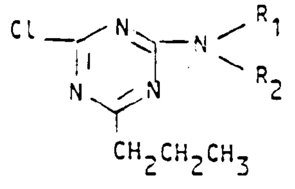

(IV)

in which $R_1$ and $R_2$ have the meaning given above, is reacted with a morpholine of the formula

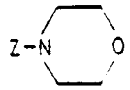

(V)

in which Z represents a hydrogen atom or a methyl radical ; or

b) in order to prepare the 2-amino-4-morpholino-6-propyl-1,3,5-triazines having the general formula I wherein $R_1$ represents a hydrogen atom, an alkyl or phenylalkyl radical and $R_2$ represents a $COR_3$ group in which $R_3$ is an alkyl, phenyl, halophenyl, alkylphenyl, alkoxyphenyl, phenylalkyl or phenoxy radical, the alkyl and alkoxy radicals having 1 to 4 carbon atoms, a 2-amino-4-morpholino-6-propyl-1,3,5-triazine of the formula

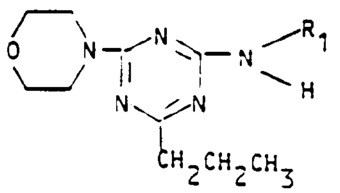

(VI)

in which $R_1$ has the meaning given above, is reacted in equimolar proportions with an $R_3$-carbonyl halide of the formula $HalCOR_3$ (VII) in which $R_3$ has the meaning given above and Hal is a halogen atom ; or

c) in order to prepare the 2-amino-4-morpholino-6-propyl-1,3,5-triazines having the general formula I wherein $R_1$ represents a hydrogen atom, an alkyl or phenylalkyl radical and $R_2$ represents an alkanoyloxyalkyl, benzoyloxyalkyl or phenylacetyloxyalkyl radical, the alkyl and alkanoyl radicals having 1 to 4 carbon atoms, a (4-morpholino-6-propyl-1,3,5-triazin-2-yl)-aminoalkanol of the formula

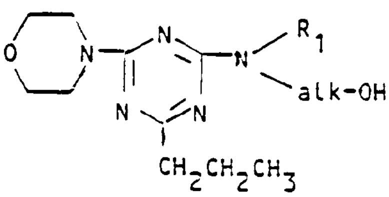

(VIII)

in which $R_1$ has the meaning given above and alk represents an alkylene radical having 1 to 4 carbon atoms, is reacted in equimolar proportions with an $R_6$-carbonyl halide of the formula $HalCOR_6$ (IX) in which $R_6$ represents an alkyl radical having 1 to 4 carbon atoms, phenyl or benzyl and Hal is a halogen atom ; or

d) in order to prepare the 2-amino-4-morpholino-6-propyl-1,3,5-triazines having the general formula I wherein $R_1$ represents a hydrogen atom, an alkyl or phenylalkyl radical and $R_2$ represents a $CONR_4R_5$ group, in which $R_4$ and $R_5$ represent hydrogen or an alkyl radical, the alkyl radicals having 1 to 4 carbon atoms, a phenyl (4-morpholino-6-propyl-1,3,5-triazin-2-yl)carbamate of the formula

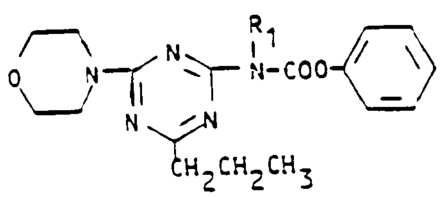

(X)

in which $R_1$ has the meaning given above, is reacted with a nitrogen compound of the formula $HNR_4R_6$ (XI) in which $R_4$ and $R_6$ have the meaning given above ; or

e) in order to prepare the 2-amino-4-morpholino-6-propyl-1,3,5-triazines having the general formula I wherein $R_1$ represents a hydrogen atom, an alkyl or phenylalkyl radical and $R_2$ represents an aminocarbonyloxyalkyl radical, the alkyl radicals having 1 to 4 carbon atoms, ammonia is reacted with a 2-morpholino-4-(phenoxycarbonyloxyalkylamino)-6-propyl-1,3,5-triazine of the formula

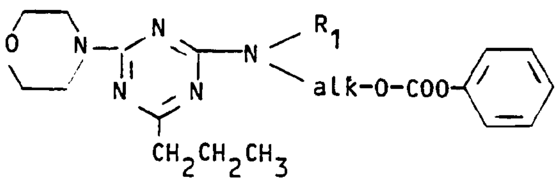

(XII)

in which $R_1$ has the meaning given above and alk represents an alkylene radical having 1 to 4 carbon atoms ; or

f) in order to prepare the 2-amino-4-morpholino-6-propyl-1,3,5-triazines having the general formula I wherein $R_1$ represents the acetyl radical and $R_2$ an acetoxyalkyl radical, the alkyl radical of which has 1 to 4 carbon atoms, at least two moles of an acetyl halide are reacted with one mole of a (4-morpholino-6-propyl-1,3,5-triazin-2-yl)aminoalkanol of the formula

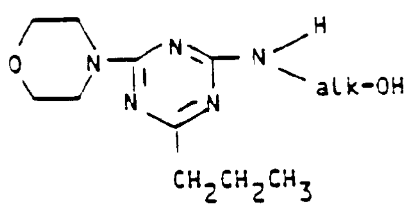

(XIV)

in which alk represents an alkylene radical having 1 to 4 carbon atoms ; and

g) optionally, the 2-amino-4-morpholino-6-propyl-1,3,5-triazines of the formula I thus obtained are converted into their non-toxic pharmaceutically acceptable acid addition salts.